# EUROPÄISCHE PATENTANMELDUNG

(11) **EP 4 682 687 A2**
(43) Veröffentlichungstag der Anmeldung: **21.01.2026**
(21) Anmeldenummer: 25221387.1
(22) Anmeldetag: 16.12.2020
(51) Int. Cl.: G06F 3/01

(54) **AUFLAGEEINRICHTUNG ZUR AUFLAGE EINES KÖRPERTEILS EINES NUTZERS**

(30) Priorität: 19.12.2019 DE 102019135329
(62) Teilanmeldung aus: 20839256.3
(71) Anmelder: CO12 GmbH, 96215 Lichtenfels (DE)
(72) Erfinder: HENZLER, Jens, 96215 Lichtenfels (DE); GÜTTLER, Ingo, 96215 Lichtenfels (DE); HOFMANN, Oliver, 96215 Lichtenfels (DE)
(74) Vertreter: Hafner & Kohl PartmbB

(57) **Zusammenfassung**

Auflageeinrichtung (1) zur zumindest abschnittsweisen, gegebenenfalls vollständigen, Auflage wenigstens eines Körperteils eines Nutzers, umfassend einen Auflagekörper (2), welcher wenigstens einen Auflagebereich (3) zur zumindest abschnittsweisen, gegebenenfalls vollständigen, Auflage wenigstens eines Körperteils eines Nutzers aufweist.

## Beschreibung

Die Erfindung betrifft eine Auflageeinrichtung zur zumindest abschnittsweisen, gegebenenfalls vollständigen, Auflage wenigstens eines Körperteils eines Nutzers bzw. einer Nutzerin, welche Auflageeinrichtung einen Auflagekörper, welcher wenigstens einen Auflagebereich zur zumindest abschnittsweisen, gegebenenfalls vollständigen, Auflage wenigstens eines Körperteils eines Nutzers aufweist, umfasst.

Entsprechende Auflageeinrichtungen sind aus dem Stand der Technik in unterschiedlichen Ausführungen an und für sich bekannt und umfassen typischerweise einen Auflagekörper, welcher wenigstens einen Auflagebereich zur zumindest abschnittsweisen, gegebenenfalls vollständigen, Auflage wenigstens eines Körperteils, wie z. B. einer Gliedmaße, eines Nutzers bzw. einer Nutzerin aufweist.

Die Hauptfunktion einer entsprechender Auflageeinrichtungen besteht in der Lagerung einer auf einem entsprechenden Auflagebereich eines Auflagekörpers der Auflageeinrichtung aufgelegten Körperteils. Der Auflagebereich soll dabei derart gestaltet sein, dass ein Körperteil in einer körperteilspezifisch möglichst entspannten Position aufgelegt bzw. gelagert werden kann; dies kann zweckmäßig sein, um, gegebenenfalls im Rahmen einer Therapie, Verspannungen zu lösen bzw. die Eigenspannung (Tonus) der Muskeln zu beeinflussen.

In diesem Zusammenhang besteht ein Verbesserungs- bzw. Weiterentwicklungsbedarf bekannter Auflageeinrichtungen, als es sich bei den diesen zugehörigen Auflagekörpern typischerweise um individuell ausgeführte Formkörper handelt, sodass der fertigungstechnische Aufwand zur Herstellung der Auflageeinrichtungen aufgrund der Vielzahl an anatomisch unterschiedlichen Körperteilen - selbst bei Betrachtung nur einer bestimmten Körperteilart, wie z. B. einer Hand - vergleichsweise hoch ist.

Der Erfindung liegt hiervon ausgehend die Aufgabe zugrunde, eine demgegenüber verbesserte Auflageeinrichtung zur Auflage eines Körperteils eines Nutzers anzugeben.

Die Aufgabe wird durch eine Auflageeinrichtung gemäß Anspruch 1 gelöst. Die hierzu abhängigen Ansprüche betreffen mögliche Ausführungsformen der Auflageeinrichtung.

Ein erster Aspekt der Erfindung betrifft eine Auflageeinrichtung zur zumindest abschnittsweisen, gegebenenfalls vollständigen, Auflage wenigstens eines Körperteils eines Nutzers bzw. einer Nutzerin. Die Auflageeinrichtung dient sonach im Allgemeinen zur zumindest abschnittsweisen, gegebenenfalls vollständigen, Auflage wenigstens eines Körperteils eines Nutzers bzw. einer Nutzerin. Wenngleich im Folgenden allein der Begriff "Nutzer" verwendet wird, ist hierunter selbstverständlich auch eine Nutzerin zu verstehen.

Der Begriff "Auflage" kann als Lagerung wenigstens eines Körperteils bzw. wenigstens eines Körperteilabschnitts verstanden werden. Die Auflageeinrichtung kann sonach auch als Lagerungseinrichtung bezeichnet bzw. erachtet werden. Der der Auflageeinrichtung zugehörige Auflagekörper kann entsprechend als Lagerungskörper bezeichnet bzw. erachtet werden. Der dem Auflagekörper zuzuordnende Auflagebereich kann entsprechend als Lagerungsbereich bezeichnet bzw. erachtet werden.

Der Begriff "Körperteil" kann sowohl als Körperteil als auch als Körperteilabschnitt verstanden werden. Ein Beispiel für ein Körperteil ist der Rumpf oder eine Gliedmaße. Ein Beispiel für einen Körperteilabschnitt ist demnach ein Rumpfabschnitt bzw. ein Gliedmaßenabschnitt.

Der Begriff "Gliedmaße" kann sowohl als komplette Gliedmaße (Extremität) als auch als ein Gliedmaßenabschnitt einer übergeordneten Gliedmaße verstanden werden. Ein Beispiel für eine Gliedmaße ist sonach ein Arm oder ein Bein. Ein Beispiel für einen Gliedmaßenabschnitt ist demnach eine Hand oder ein Fuß. Eine Hand oder ein Fuß kann jedoch auch ein Beispiel für eine Gliedmaße sein. Ein Beispiel für einen Gliedmaßenabschnitt kann demnach auch ein Finger oder ein Zeh sein.

Aus vorstehenden Ausführungen ergibt sich, dass die Auflageeinrichtung insbesondere zur Auflage einer Gliedmaße einer oberen Extremität eines Nutzers, insbesondere einer Hand, eines Handabschnitts, eines Arms oder eines Armabschnitts, oder zur Auflage einer Gliedmaße einer unteren Extremität eines Nutzers, insbesondere eines Fußes, eines Fußabschnitts, eines Beins oder eines Beinabschnitts, eingerichtet sein kann.

Die Auflageeinrichtung dient typischerweise zur Auflage bzw. Lagerung von Körperteilen, d. h. insbesondere Gliedmaßen, bzw. Körperteilabschnitten, d. h. insbesondere Gliedmaßenabschnitten, des Menschen bzw. menschlichen Körpers; die Auflage bzw. Lagerung von Körperteilen bzw. Körperteilabschnitten eines Tiers bzw. eines tierischen Körpers sind jedoch ebenso denkbar.

Die Auflageeinrichtung umfasst, wie erwähnt, einen Auflagekörper. Der, wie ebenso erwähnt, auch als Lagerungskörper zu bezeichnende bzw. zu erachtende Auflagekörper weist wenigstens einen, wie ebenso erwähnt, auch als Lagerungsbereich zu bezeichnenden bzw. zu erachtenden Auflagebereich zur zumindest abschnittsweisen, gegebenenfalls vollständigen, Auflage wenigstens eines Körperteils, d. h. z. B. einer Gliedmaße, eines Nutzers auf. Der Auflagebereich ist sonach derjenige Bereich der Auflageeinrichtung, auf welchem ein Nutzer bei bestimmungsgemäßer Nutzung der Auflageeinrichtung ein Körperteil (direkt) auflegt. Der Auflagebereich stellt sonach den eigentlichen Bereich der Auflageeinrichtung, auf welchem ein Nutzer bei bestimmungsgemäßer Nutzung der Auflageeinrichtung ein Körperteil auflegt bzw. lagert, dar. Der Auflagebereich bildet sonach typischerweise zumindest einen Teil der (freiliegenden) Oberfläche der Auflageeinrichtung.

Die Auflageeinrichtung umfasst sonach einen, z. B. blockartig bzw. -förmig vordefiniert konfiguriert ausgebildeten, Auflagekörper, welcher wenigstens eine Oberfläche aufweist, die zumindest abschnittsweise, gegebenenfalls vollständig, eine im Hinblick auf ein bestimmtes Körperteil eines bestimmten Nutzers vordefiniert konfiguriert ausgebildete dreidimensionale Konturierung bzw. Strukturierung aufweist. Diese dreidimensionale Konturierung bzw. Strukturierung ist dabei typischerweise durch den Auflagebereich gebildet. Der Auflagebereich kann dabei einstückig mit dem Auflagekörper ausgebildet sein. Ebenso ist es denkbar, dass der Auflagebereich als ein gesondertes Bauteil ausgebildet ist, welches mit dem Auflagekörper unter Ausbildung der Auflageeinrichtung verbindbar oder verbunden ist.

Die Abmessungen der Auflageeinrichtung respektive des Auflagekörpers respektive des Auflagebereichs sind typischerweise an die Abmessungen des jeweils aufzulegenden Körperteils angepasst. Bei bestimmungsgemäßer Nutzung der Auflageeinrichtung ist der Auflagebereich durch den jeweils aufgelegten Körperteil sonach zumindest abschnittsweise, gegebenenfalls vollständig, abgedeckt.

Die Auflageeinrichtung zeichnet sich dadurch aus, dass zumindest der Auflagebereich zumindest abschnittsweise, gegebenenfalls vollständig, eine auf Grundlage einer Körperteilabmessungsinformation, welche die Abmessungen eines auf die Auflageeinrichtung aufzulegenden Körperteils zumindest abschnittsweise, gegebenenfalls vollständig, beschreibt, aus einer Mehrzahl an vordefinierten Auflageeinrichtungsabmessungsinformationen, wobei jede Auflageeinrichtungsabmessungsinformation wenigstens einer Abmessung eines Körperteils zugeordnete vordefinierte Abmessungen einer auf Grundlage der jeweiligen Auflageeinrichtungsabmessungsinformation herstellbaren Auflageeinrichtung beschreibt, ausgewählten Auflageeinrichtungsabmessungsinformation beschreibenden Daten vordefiniert konfiguriert ausgebildete geometrisch-konstruktive Gestaltung bzw. Formgebung aufweist. Der Auflagebereich weist sonach eine vordefiniert konfigurierte geometrisch-konstruktive Gestaltung bzw. Formgebung auf, welche auf Grundlage von durch eine Auflageeinrichtungsabmessungsinformation beschriebenen Daten ausgebildet bzw. erzeugt ist.

Die die Grundlage der für die Ausbildung bzw. Erzeugung des Auflagebereichs verwendeten Daten bildende Auflageeinrichtungsabmessungsinformation ist aus einer Mehrzahl an vordefinierten Auflageeinrichtungsabmessungsinformationen, wobei jede Auflageeinrichtungsabmessungsinformation wenigstens einer Abmessung, wie z. B. Länge, Breite, Höhe, etc., oder wenigstens eines Abmessungsverhältnisses, wie z. B. eines Länge-Breite-Verhältnisses, eines Länge-Höhe-Verhältnisses, eines Breite-Höhe-Verhältnisses, eines Länge-Breite-Höhe-Verhältnisses, etc., eines Körperteils zugeordnete vordefinierte Abmessungen einer auf Grundlage der jeweiligen Auflageeinrichtungsabmessungsinformation herstellbaren Auflageeinrichtung beschreibt, ausgewählt. Die Auswahl der Auflageeinrichtungsabmessungsinformation aus der Mehrzahl an Auflageeinrichtungsabmessungsinformationen ist auf Grundlage einer Körperteilabmessungsinformation, welche die Abmessungen eines auf die Auflageeinrichtung aufzulegenden Körperteils zumindest abschnittsweise, gegebenenfalls vollständig, beschreibt, erfolgt.

Die vordefiniert konfigurierte geometrisch-konstruktive Gestaltung bzw. Formgebung des Auflagebereichs eröffnet erhebliche fertigungstechnische Vorteile, als es sich bei dem Auflagebereich und typischerweise auch der gesamten Auflageeinrichtung nicht um einen individuell konfiguriert ausgebildeten Bauteilabschnitt bzw. nicht um ein individuell konfiguriert ausgebildetes Bauteil handelt. Der Auflagebereich ist nämlich vordefiniert konfiguriert, d. h. er weist eine konkrete vordefiniert konfigurierte räumlich-körperliche Gestaltung und somit eine konkrete vordefiniert konfigurierte Raumform auf, welche, wenngleich diese typischerweise in abstrahierter bzw. standardisierter und somit ebenso in vordefiniert konfigurierter Form, im Hinblick auf gewisse Merkmale eines jeweilig aufzulegenden Körperteils, wie z. B. Anzahl und Anordnung von Fingern einer (menschlichen) Hand, Anzahl und Anordnung von Zehen eines (menschlichen) Fußes, etc., ausgebildete Auflageabschnitte aufweist, nicht im Hinblick auf ein auf der Auflageeinrichtung aufzulegendes Körperteil individualisiert ist. Analoges gilt für die gesamte Auflageeinrichtung; dies insbesondere dann, wenn der Auflagebereich einstückig mit dem Auflagekörper ausgebildet ist.

Der Auflagebereich bzw. die Auflageeinrichtung weist sonach keine individuell konfiguriert ausgebildete geometrisch-konstruktive Gestaltung und somit, abgesehen von einer etwaigen Anpassung bzw. Skalierung der Abmessungen, keine Individualisierung auf. Bei dem Auflagebereich bzw. der Auflageeinrichtung handelt es sonach nicht um ein für ein bestimmtes Körperteil eines bestimmten Nutzers individuell konfiguriertes Einzelteil. Vielmehr kann der Auflagebereich bzw. die Auflageeinrichtung als ein im Hinblick auf bestimmte Abmessungen eines Körperteils, jedoch im Übrigen völlig unabhängig von individuellen Merkmalen des jeweiligen Körperteils, als ein fertigungstechnisch einfach herstellbares Massenbauteil verstanden werden. Der Auflagebereich bzw. die Auflageeinrichtung weist aufgrund der vordefiniert konfigurierten geometrisch-konstruktiven Gestaltung bzw. Formgebung typischerweise eine zumindest abschnittsweise, gegebenenfalls vollständig, standardisierte geometrisch-konstruktive Gestaltung bzw. Formgebung auf, welche, wenn überhaupt, allein durch Skalierung im Hinblick auf die Abmessungen eines konkret aufzulegenden Körperteils angepasst ist bzw. wird.

Die vordefiniert konfigurierte geometrisch-konstruktive Gestaltung des Auflagebereichs - hierunter ist insbesondere eine vordefiniert konfigurierte dreidimensionale Konturierung bzw. Strukturierung der Oberfläche des Auflagebereichs zu verstehen - stellt typischerweise ein abstrahiert bzw. standardisiert konfiguriertes (negatives) Abbild eines auf dem Auflagebereich aufzulegenden Körperteils dar. Die Abstrahierung bzw. Standardisierung resultiert - trotz dessen es sich nicht um eine nutzerspezifisch bzw. körperteilspezifisch individuell konfigurierte Passform der Auflageeinrichtung handelt - typischerweise in einer ausreichend genauen Passform des Auflagebereichs, welche eine Auflage eines jeweiligen Körperteils auf dem Auflagebereich in einer körperteilspezifisch entspannenden bzw. entspannten Position ermöglicht.

Eine Auflage eines Körperteils in einer körperteilspezifisch entspannenden bzw. entspannten Position kann sowohl einen Entspannungseffekt auf das jeweilige Körperteil als auch auf an das jeweilige Körperteil anschließende weitere Körperteile bewirken, sodass ein Nutzer bei Benutzung der Auflageeinrichtung insgesamt in einen entspannenden bzw. entspannten Zustand gebracht werden kann. Neben dem reinen Entspannungseffekt respektive damit einhergehend können, soweit erforderlich, auch Therapieeffekte bewirkt werden, als durch die Auflage des Körperteils in einer körperteilspezifisch entspannenden bzw. entspannten Position z. B. die Durchblutung und/oder die Sauerstoffversorgung des jeweiligen Körperteils therapeutisch wirksam beeinflusst werden kann. Positive Effekte können insbesondere bei rheumatischen Körperteilen bzw. Gliedmaßen und/oder bei, z. B. aufgrund eines Schlaganfalls, spastischen Körperteilen bzw. Gliedmaßen erreicht werden. Die mit der Spastik einhergehende Erhöhung der Eigenspannung (Tonus) der Muskulatur des jeweiligen Körperteils kann durch (regelmäßige) Benutzung der Auflageeinrichtung (teilweise deutlich) reduziert werden.

Die Auflageeinrichtung kann dabei insbesondere ohne Hilfestellung durch einen Dritten benutzt werden; mithin kann der Nutzer im Sinne einer Selbsthilfe durch bestimmungsgemäße Benutzung der Auflageeinrichtung selbstständig therapeutische Maßnahmen, z. B. zur Tonusregulation, Schmerztherapie, etc., vornehmen bzw. sich auf entsprechende therapeutische Maßnahmen vorbereiten.

Für das Beispiel einer spastischen Hand ermöglicht es die insoweit auch als Handballenauflage für die Therapie von Personen mit einer krampfenden oder verkrampften Hand zu bezeichnende bzw. zu erachtende Auflageeinrichtung, dass die Hand auf der Auflageeinrichtung in einem entkrampften Zustand aufliegt, wodurch ein Entspannungszustand erreicht wird.

Wie erwähnt, kann der Auflagebereich einstückig mit dem Auflagekörper ausgebildet sein. In dieser ersten Variante bildet der Auflagebereich typischerweise eine (freiliegende) Oberfläche des Auflagekörpers.

Wie ebenso erwähnt, ist es jedoch ebenso denkbar, dass der Auflagebereich als zu dem Auflagekörper gesondertes Bauteil ausgebildet und mit dem Auflagekörper bedarfsweise unter Ausbildung der Auflageeinrichtung, gegebenenfalls (beschädigungs- bzw. zerstörungsfrei) lösbar, verbindbar oder verbunden ist. Diese zweite Variante bedingt keine fertigungstechnischen Nachteile, als sowohl der Auflagekörper als auch der Auflagebereich standardisiert ausgeführt hergestellt werden können.

In der zweiten Variante kann der Auflagekörper und/oder der Auflagebereich geeignete, gegebenenfalls korrespondierende, Verbindungselemente umfassen, welche eine stabile, gleichwohl typischerweise (beschädigungs- bzw. zerstörungsfrei) lösbare, Verbindung des Auflagekörpers mit dem Auflagebereich, und umgekehrt, ermöglichen. Der Auflagekörper kann z. B. wenigstens ein erstes Verbindungselement und der Auflagebereich wenigstens ein zweites Verbindungselement aufweisen, wobei das wenigstens eine erste und das wenigstens eine zweite Verbindungselement eingerichtet sind, miteinander unter Ausbildung der Auflageeinrichtung zusammenzuwirken. Das wenigstens eine erste Verbindungselement und das wenigstens eine zweite Verbindungselement können z. B. eingerichtet sein, miteinander unter Ausbildung der Auflageeinrichtung form- und/oder kraftschlüssig zusammenzuwirken. Alternativ oder ergänzend ist es auch denkbar, dass das wenigstens eine erste Verbindungselement und das wenigstens eine zweite Verbindungselement eingerichtet sind, miteinander unter Ausbildung der Auflageeinrichtung magnetisch zusammenzuwirken.

Der Auflagebereich kann eine oder mehrere, insbesondere als Aufnahmen ausgebildete, Auflageabschnitte, welche zur Aufnahme eines bestimmten Körperteilabschnitts, insbesondere eines Gliedmaßenabschnitts, beispielsweise eines Fingers oder einer Zehe, eines auf dem Auflagebereich aufzulegenden bzw. aufgelegten Körperteils, d. h. insbesondere einer Gliedmaße, eingerichtet sind, aufweisen. Eine für die Auflage eines bestimmten Körperteils vorgesehene Auflageeinrichtung kann sonach eine Anzahl an körperteilspezifisch vordefiniert konfiguriert ausgebildeten Auflageabschnitten und somit körperteilspezifisch standardisiert konfiguriert ausgebildeten Auflageabschnitten aufweisen. Jeweilige Auflageabschnitte können z. B. als vordefiniert bzw. standardisiert konfigurierte Aufnahmen ausgebildet sein. Eine jeweilige vordefiniert bzw. standardisiert konfigurierte Aufnahme kann zur Aufnahme eines oder mehrerer Körperteile ausgebildet sein. Typischerweise ist eine jeweilige vordefiniert bzw. standardisiert konfigurierte Aufnahme zur Aufnahme (genau) eines bestimmten Körperteils ausgebildet.

Der Auflagekörper oder der Auflagebereich kann eine bestimmte Anzahl und/oder eine bestimmte Anordnung an entsprechenden vordefiniert bzw. standardisiert konfiguriert ausgebildeten Auflageabschnitten aufweisen, welche der Anzahl und/oder Anordnung der Gliedmaßenabschnitte, d. h. z. B. der Finger oder Zehen, eines jeweiligen auf dem Auflagebereich aufzulegenden Körperteils entspricht. Für das Beispiel einer auf dem Auflagebereich aufzulegenden Hand, kann der Auflagekörper bzw. der Auflagebereich sonach z. B. mehrere Auflageabschnitte aufweisen, wobei jeder Auflagebereichsabschnitt der Auflage eines bestimmten Fingers der aufzulegenden Hand dient. Analoges gilt für andere Körperteile.

Der Auflagekörper oder der Auflagebereich können insbesondere eine bestimmte Anzahl und/oder eine bestimmte Anordnung an vordefiniert konfiguriert ausgebildeten Auflageabschnitten aufweisen, welche der Anzahl und/oder Anordnung der Gliedmaßenabschnitte, insbesondere der Finger oder Zehen, eines jeweiligen auf der Auflageeinrichtung aufzulegenden Körperteils eines Nutzers entspricht.

Entsprechende Auflageabschnitte können, wenn sie der jeweiligen Auflage eines Fingers dienen, als Fingerauflageabschnitte und wenn sie der jeweiligen Auflage einer Zehe dienen, als Zehenauflageabschnitte bezeichnet bzw. erachtet werden.

Für die Auflage einer Hand kann der Auflagekörper oder der Auflagebereich konkret sonach wenigstens zwei, insbesondere wenigstens drei, insbesondere wenigstens vier, gegebenenfalls fünf, vordefiniert konfiguriert ausgebildete Fingerauflageabschnitte in paralleler Anordnung, insbesondere in im Wesentlichen paralleler Anordnung, aufweisen, welche der Anzahl und Anordnung der Finger einer jeweiligen auf der Auflageeinrichtung aufzulegenden Hand eines Nutzers entspricht. Derart ist ein besonders entspannender Effekt auf eine auf der Auflageeinrichtung aufzulegende bzw. aufgelegte Hand erzielbar. Jeweilige Fingerauflageabschnitte sind ebenso vordefiniert konfiguriert bzw. standardisiert ausgebildet. Jeweilige Fingerauflageabschnitte können konkret z. B. als vordefiniert bzw. standardisiert konfiguriert ausgebildete Aufnahmen bzw. Vertiefungen ausgebildet sein.

Jeweilige Fingerauflageabschnitte können sich in einer ersten Raumrichtung, insbesondere einer distalen Raumrichtung bezogen auf eine bzw. die auf der Auflageeinrichtung aufzulegende bzw. aufgelegte Hand, ersteckend angeordnet und/oder ausgerichtet sein. Derart ist ein besonders entspannender Effekt auf eine auf der Auflageeinrichtung aufzulegende bzw. aufgelegte Hand erzielbar. Analoges gilt für andere Körperteile.

Die Auflageeinrichtung kann ferner wenigstens einen, insbesondere sich quer zu den Fingerauflageabschnitten erstreckend angeordneten oder ausgerichteten, weiteren Auflageabschnitt zur Aufnahme eines Endbereichs eines Mittelhandbereichs, d. h. insbesondere eines proximalen Endbereichs, einer auf der Auflageeinrichtung aufzulegenden Hand eines Nutzers aufweisen bzw. umfassen. Derart ist ein besonders entspannender Effekt auf eine auf der Auflageeinrichtung aufzulegende bzw. aufgelegte Hand erzielbar. Ein entsprechender weiterer Auflageabschnitt ist ebenso vordefiniert konfiguriert bzw. standardisiert ausgebildet. Ein entsprechender weiterer Auflageabschnitt kann konkret z. B. als vordefiniert bzw. standardisiert konfiguriert ausgebildete Aufnahme bzw. Vertiefung ausgebildet sein.

Jeweilige Fingerauflageabschnitte können, insbesondere unmittelbar, in den weiteren Auflageabschnitt übergehen. Jeweilige Fingerauflageabschnitte können sonach, insbesondere unmittelbar, mit dem weiteren Auflageabschnitt kommunizieren. Derart ist ebenso ein besonders entspannender Effekt auf eine auf der Auflageeinrichtung aufzulegende bzw. aufgelegte Hand erzielbar.

Der Auflagekörper oder der Auflagebereich können einen Grundabschnitt aufweisen bzw. umfassen, welcher eine oder mehrere, insbesondere relativ zu einer ungewölbten Grundebene, insbesondere einer Hand nachempfunden, gewölbt ausgebildete Wölbungsabschnitte aufweist. Jeweilige Wölbungsabschnitte sind ebenso vordefiniert konfiguriert bzw. standardisiert ausgebildet. Jeweilige Wölbungsabschnitte können konkret z. B. als vordefiniert bzw. standardisiert konfiguriert ausgebildete, insbesondere pelottenartige bzw. -förmige, Erhöhungen ausgebildet sein. Derart ist ebenso ein besonders entspannender Effekt auf eine auf der Auflageeinrichtung aufzulegende bzw. aufgelegte Hand erzielbar.

Der Grundabschnitt kann insbesondere wenigstens einen, insbesondere pelottenartigen bzw. - förmigen, Wölbungsabschnitt aufweisen, welcher im bestimmungsgemäß genutzten Zustand der Auflageeinrichtung zur Stützung eines Mittelhandbereichs einer auf der Auflageeinrichtung aufzulegenden oder aufgelegten Hand eines Nutzers eingerichtet ist. Derart ist ebenso ein besonders entspannender Effekt auf eine auf der Auflageeinrichtung aufzulegende bzw. aufgelegte Hand erzielbar.

Jeweilige Fingerauflageabschnitte und/oder der wenigstens eine weitere Auflageabschnitt können z. B. in wenigstens einem, insbesondere ringsegmentartig oder -förmig, gewölbt ausgebildeten Wölbungsabschnitt ausgebildet sein. Derart ist ebenso ein besonders entspannender Effekt auf eine auf der Auflageeinrichtung aufzulegende bzw. aufgelegte Hand erzielbar.

Unabhängig von der Anzahl und/oder Anordnung jeweiliger Auflageabschnitte kann der Auflagekörper oder der Auflagebereich eine einem auf der Auflageeinrichtung aufzulegenden Körperteil nachempfunden vordefiniert konfiguriert ausgebildete Grundform aufweisen. Dies kann sich positiv auf das Nutzungsverhalten auswirken, als die Grundform des Auflagekörpers bzw. des Auflagebereichs bereits intuitiv erfassbar zum Auflegen des jeweiligen Körperteils "einlädt"; die Grundform des Auflagekörpers bzw. des Auflagebereichs kann sich sonach positiv auf die "Nutzer-Compliance" auswirken.

Der Auflagekörper kann auf einer Grund- oder Trägerplatte angeordnet oder ausgebildet sein. Die Ausbildung bzw. Anordnung des Auflagekörpers auf einer Grund- bzw. Trägerplatte stellt eine stabile Auflage bzw. Lagerung der Auflageeinrichtung auf einem Untergrund sicher. Ein entsprechende Grund- bzw. Trägerplatte kann zudem zumindest abschnittsweise, gegebenenfalls vollständig, mit wenigstens einer haftungssteigernden Strukturierung, wie z. B. einer besonderen Oberflächenstrukturierung, z. B. einer Aufrauung, einer Gummierung, etc., versehen sein, um eine stabile Auflage bzw. Lagerung der Auflageeinrichtung auf einem Untergrund sicherzustellen.

Es ist auch denkbar, dass eine entsprechende Grund- bzw. Trägerplatte einen Bestandteil einer Trägereinrichtung zum Tragen der Auflageeinrichtung bildet. Eine entsprechende Grund- bzw. Trägerplatte kann sonach nebst darauf angeordnetem oder ausgebildetem Auflagekörper, z. B. durch Auf- oder Einsetzen, mit einem, z. B. rahmenartigen bzw. -förmigen, Trägerelement bedarfsweise unter Ausbildung einer entsprechenden Trägereinrichtung, gegebenenfalls (beschädigungs- bzw. zerstörungsfrei) lösbar, verbindbar oder verbunden sein. Die Grund- bzw.

Trägerplatte bzw. das Trägerelement können geeignete, gegebenenfalls korrespondierende, Verbindungselemente umfassen, welche eine stabile, gleichwohl typischerweise (beschädigungs- bzw. zerstörungsfrei) lösbare, Verbindung der Grund- bzw. Trägerplatte mit dem Trägerelement, und umgekehrt, ermöglichen. Die Grund- bzw. Trägerplatte kann z. B. wenigstens ein erstes Verbindungselement und das Trägerelement wenigstens ein zweites Verbindungselement aufweisen, wobei das wenigstens eine erste und das wenigstens eine zweite Verbindungselement eingerichtet sind, miteinander unter Ausbildung der Trägereinrichtung zusammenzuwirken. Das wenigstens eine erste Verbindungselement und das wenigstens eine zweite Verbindungselement können z. B. eingerichtet sein, miteinander unter Ausbildung der Trägereinrichtung form- und/oder kraftschlüssig zusammenzuwirken. Alternativ oder ergänzend ist es auch denkbar, dass das wenigstens eine erste Verbindungselement und das wenigstens eine zweite Verbindungselement eingerichtet sind, miteinander unter Ausbildung der Trägereinrichtung magnetisch zusammenzuwirken.

Die Auflageeinrichtung kann eine, insbesondere in den Auflagekörper und/oder in den Auflagebereich integrierte, Erfassungseinrichtung umfassen, welche zur Erfassung wenigstens einer chemischen und/oder physikalischen Größe des Auflagekörpers und/oder des Auflagebereichs und/oder eines auf dem Auflagebereich aufgelegten Körperteils eingerichtet ist. Eine entsprechende Erfassungseinrichtung kann z. B. zur Erfassung chemischer Parameter, wie z. B. der Zusammensetzung der Oberfläche eines aufgelegten Körperteils oder einer Substanz auf der Oberfläche eines aufgelegten Körperteils, und/oder thermischer Parameter, wie z. B. der Temperatur des Auflagekörpers und/oder eines aufgelegten Körperteils, und/oder mechanischer Parameter, wie z. B. von, insbesondere vermittels eines aufgelegten Körperteils, auf den Auflagekörper und/oder den Auflagebereich ausgeübten, Kräften, d. h. insbesondere Gewichtskräften, Druck, Spannung, etc., und/oder hydrometrischer Parameter, wie z. B. der Feuchtigkeit eines aufgelegten Körperteils, etc. eingerichtet sein. Eine entsprechende Erfassungseinrichtung kann ein oder mehrere, z. B. als Sensoren ausgebildete oder solche umfassende, Erfassungselemente umfassen, welche an oder in dem Auflagekörper und/oder an oder in dem Auflagebereich angeordnet oder ausgebildet sind. Die konkrete Ausgestaltung jeweiliger Erfassungselemente richtet sich nach der jeweils zu erfassenden chemischen und/oder physikalischen Größe. Eine entsprechende Erfassungseinrichtung verfügt typischerweise über geeignete hardware- und/oder softwaremäßig implementierte Auswertungs- und/oder Verarbeitungsvorkehrungen, d. h. z. B. über geeignete Auswertungs- und/oder Verarbeitungsalgorithmen, welche eine datenmäßige Auswertung bzw. Verarbeitung jeweiliger erfassungselementseitig gelieferter Erfassungssignale ermöglichen.

Die Auflageeinrichtung kann ferner eine der Erfassungseinrichtung zuordenbare oder zugeordnete, insbesondere in den Auflagekörper und/oder in den Auflagebereich integrierte, Übertragungseinrichtung umfassen, welche zur Übertragung einer eine vermittels der Erfassungseinrichtung erfasste chemische und/oder physikalische Größe beschreibenden Erfassungsinformation an wenigstens einen Kommunikationspartner eingerichtet ist. Eine entsprechende Übertragungseinrichtung kann entsprechende Erfassungsinformationen kabelgebunden oder kabellos an wenigstens einen Kommunikationspartner übertragen. Eine kabellose Übertragung kann z. B. vermittels einer Drahtlosverbindung bzw. eines Drahtlosnetzwerks über bekannte Industriestandards, wie z. B. Bluetooth, für die Datenübertragung erfolgen. Entsprechende Erfassungsinformationen können dabei unverschlüsselt oder verschlüsselt übertragen werden. Bei einem Kommunikationspartner kann es sich z. B. um ein mobiles Endgerät, wie z. B. einen mobilen Computer (Laptop), ein Smartphone, ein Tablet, eine Smartbrille, um ein stationäres Endgerät, wie z. B. einen stationären Computer (Desktop, Server), um eine Netzwerkanwendung bzw. um einen Netzwerkspeicher (Cloud), etc. handeln.

Die Auflageeinrichtung kann ferner eine, insbesondere in den Auflagekörper und/oder in den Auflagebereich integrierte, Erfassungseinrichtung umfassen, welche zur Erfassung eines auf den Auflagebereich aufgelegten Körperteils eingerichtet ist. Eine entsprechende Erfassungseinrichtung kann z. B. zur Erfassung einer Annäherung bzw. eines Kontakts eines auf den Auflagebereich aufzulegenden bzw. aufgelegten Körperteils an den bzw. mit dem Auflagekörper bzw. an den bzw. mit dem Auflagebereich eingerichtet sein. Eine entsprechende Erfassungseinrichtung kann ein oder mehrere, z. B. als Sensoren, insbesondere Näherungs- bzw. Kontaktsensoren, ausgebildete oder solche umfassende, Erfassungselemente umfassen, welche an oder in dem Auflagekörper und/oder an oder in dem Auflagebereich angeordnet oder ausgebildet sind. Eine entsprechende Erfassungseinrichtung verfügt typischerweise über geeignete hardware- und/oder softwaremäßig implementierte Auswertungs- und/oder Verarbeitungsvorkehrungen, d. h. z. B. über geeignete Auswertungs- und/oder Verarbeitungsalgorithmen, welche eine datenmäßige Auswertung bzw. Verarbeitung jeweiliger erfassungselementseitig gelieferter Erfassungssignale ermöglichen. Eine entsprechende Erfassungseinrichtung kann jedoch auch identisch mit der bereits erwähnten Erfassungseinrichtung zur Erfassung einer chemischen und/oder physikalischen Größe eingerichtet sein, als anhand jeweiliger erfasster chemischer und/oder physikalischer Größen gegebenenfalls Rückschlüsse auf ein auf den Auflagebereich aufgelegtes Körperteil ziehbar sind.

Die Auflageeinrichtung kann ferner eine der Erfassungseinrichtung zuordenbare oder zugeordnete, insbesondere in den Auflagekörper und/oder in den Auflagebereich integrierte, Übertragungseinrichtung umfassen, welche zur Übertragung einer ein vermittels der Erfassungseinrichtung erfasstes auf den Auflagebereich aufgelegtes Körperteil beschreibenden Erfassungsinformation an wenigstens einen Kommunikationspartner eingerichtet ist. Eine entsprechende Übertragungseinrichtung kann entsprechende Erfassungsinformationen kabelgebunden oder kabellos an wenigstens einen Kommunikationspartner übertragen. Eine kabellose Übertragung kann z. B. vermittels einer Drahtlosverbindung bzw. eines Drahtlosnetzwerks über bekannte Industriestandards, wie z. B. Bluetooth, für die Datenübertragung erfolgen. Entsprechende Erfassungsinformationen können dabei unverschlüsselt oder verschlüsselt übertragen werden. Bei einem Kommunikationspartner kann es sich z. B. um ein mobiles Endgerät, wie z. B. einen mobilen Computer (Laptop), ein Smartphone, ein Tablet, eine Smartbrille, um ein stationäres Endgerät, wie z. B. einen stationären Computer (Desktop, Server), um eine Netzwerkanwendung bzw. um einen Netzwerkspeicher (Cloud), etc. handeln.

Die Auflageeinrichtung kann ferner eine, insbesondere in den Auflagekörper und/oder in den Auflagebereich integrierte, Erfassungseinrichtung umfassen, welche zur Erfassung wenigstens einer physiologischen Größe, insbesondere des Blutdrucks, der Sauerstoffsättigung oder des Pulses, eines ein Körperteil auf den Auflagebereich auflegenden Nutzers eingerichtet ist. Eine entsprechende Erfassungseinrichtung kann ein oder mehrere, z. B. als Sensoren, insbesondere Blutdruck- bzw. Sauerstoffsättigungs- bzw. Pulssensoren, ausgebildete oder solche umfassende, Erfassungselemente umfassen, welche an oder in dem Auflagekörper und/oder an oder in dem Auflagebereich angeordnet oder ausgebildet sind. Entsprechende physiologische Größen können z. B. über den Kontaktbereich zwischen dem Auflagebereich und dem jeweiligen auf den Auflagebereich aufgelegten Körperteil erfasst werden. Eine entsprechende Erfassungseinrichtung verfügt typischerweise über geeignete hardware- und/oder softwaremäßig implementierte Auswertungs- und/oder Verarbeitungsvorkehrungen, d. h. z. B. über geeignete Auswertungs- und/oder Verarbeitungsalgorithmen, welche eine datenmäßige Auswertung bzw. Verarbeitung jeweiliger erfassungselementseitig gelieferter Erfassungssignale ermöglichen.

Die Auflageeinrichtung kann ferner eine der Erfassungseinrichtung zuordenbare oder zugeordnete, insbesondere in den Auflagekörper und/oder in den Auflagebereich integrierte, Übertragungseinrichtung umfassen, welche zur Übertragung einer eine vermittels der Erfassungseinrichtung erfasste physiologische Größe beschreibenden Erfassungsinformation an wenigstens einen Kommunikationspartner eingerichtet ist. Eine entsprechende Übertragungseinrichtung kann entsprechende Erfassungsinformationen kabelgebunden oder kabellos an wenigstens einen Kommunikationspartner übertragen. Eine kabellose Übertragung kann z. B. vermittels einer Drahtlosverbindung bzw. eines Drahtlosnetzwerks über bekannte Industriestandards, wie z. B. Bluetooth, für die Datenübertragung erfolgen. Entsprechende Erfassungsinformationen können dabei unverschlüsselt oder verschlüsselt übertragen werden. Bei einem Kommunikationspartner kann es sich z. B. um ein mobiles Endgerät, wie z. B. einen mobilen Computer (Laptop), ein Smartphone, ein Tablet, eine Smartbrille, um ein stationäres Endgerät, wie z. B. einen stationären Computer (Desktop, Server), um eine Netzwerkanwendung bzw. um einen Netzwerkspeicher (Cloud), etc. handeln.

Die Auflageeinrichtung kann ferner eine Erfassungseinrichtung umfassen, welche zur Erfassung einer Bewegung des Auflagekörpers in wenigstens einem Bewegungsfreiheitsgrad, insbesondere relativ zu einem Drittgegenstand und/oder einem relativ zu einem Untergrund, und/oder zur Erfassung einer Bewegung eines auf dem Auflagebereich aufgelegten Körperteils in wenigstens einem Bewegungsfreiheitsgrad, insbesondere relativ zu dem Auflagekörper, eingerichtet ist. Eine entsprechende Erfassungseinrichtung kann ein oder mehrere, z. B. als Sensoren, insbesondere Bewegungssensoren, ausgebildete oder solche umfassende, Erfassungselemente umfassen, welche an oder in dem Auflagekörper und/oder an oder in dem Auflagebereich angeordnet oder ausgebildet sind. Eine entsprechende Erfassungseinrichtung verfügt typischerweise über geeignete hardware- und/oder softwaremäßig implementierte Auswertungs- und/oder Verarbeitungsvorkehrungen, d. h. z. B. über geeignete Auswertungs- und/oder Verarbeitungsalgorithmen, welche eine datenmäßige Auswertung bzw. Verarbeitung jeweiliger erfassungselementseitig gelieferter Erfassungssignale ermöglichen.

Die Auflageeinrichtung kann ferner eine der Erfassungseinrichtung zuordenbare oder zugeordnete, insbesondere in den Auflagekörper und/oder in den Auflagebereich integrierte, Übertragungseinrichtung, welche zur Übertragung einer eine vermittels der Erfassungseinrichtung erfassten Bewegung des Auflagekörpers in wenigstens einem Bewegungsfreiheitsgrad, insbesondere relativ zu einem Untergrund, und/oder einer vermittels der Erfassungseinrichtung erfassten Bewegung eines auf dem Auflagekörper aufgelegten Gliedmaße in wenigstens einem Bewegungsfreiheitsgrad, insbesondere relativ zu dem Auflagekörper an wenigstens einen Kommunikationspartner eingerichtet ist. Eine entsprechende Übertragungseinrichtung kann entsprechende Erfassungsinformationen kabelgebunden oder kabellos an wenigstens einen Kommunikationspartner übertragen. Eine kabellose Übertragung kann z. B. vermittels einer Drahtlosverbindung bzw. eines Drahtlosnetzwerks über bekannte Industriestandards, wie z. B. Bluetooth, für die Datenübertragung erfolgen. Entsprechende Erfassungsinformationen können dabei unverschlüsselt oder verschlüsselt übertragen werden. Bei einem Kommunikationspartner kann es sich z. B. um ein mobiles Endgerät, wie z. B. einen mobilen Computer (Laptop), ein Smartphone, ein Tablet, eine Smartbrille, um ein stationäres Endgerät, wie z. B. einen stationären Computer (Desktop, Server), um eine Netzwerkanwendung bzw. um einen Netzwerkspeicher (Cloud), etc. handeln.

Die Auflageeinrichtung kann ferner eine, insbesondere in den Auflagekörper und/oder in den Auflagebereich integrierte, Datenspeichereinrichtung umfassen, welche zur datenmäßigen Speicherung wenigstens einer vermittels der Erfassungseinrichtung erfassten Erfassungsinformation eingerichtet ist. Einer entsprechenden Datenspeichereinrichtung ist typischerweise eine Datenübertragungsschnittstelle zugeordnet, über welche Daten kabelgebunden oder kabellos von der Datenspeichereinrichtung gelesen und/oder über welche Daten in die Datenspeichereinrichtung geschrieben werden können.

Die Auflageeinrichtung kann ferner eine, insbesondere in den Auflagekörper und/oder in den Auflagebereich integrierte, Signalausgabeeinrichtung umfassen, welche, insbesondere auf Grundlage wenigstens einer seitens der oder einer Erfassungseinrichtung erzeugten Erfassungsinformation, zur Erzeugung wenigstens eines, insbesondere akustischen und/oder optischen und/oder haptischen, Ausgabesignals eingerichtet ist. Über eine entsprechende Signalausgabeeinrichtung können vermittels der Auflageeinrichtung sonach bestimmte Ausgabesignale, d. h. allgemein Signale, ausgegeben werden. Beispielsweise kann ein ein ordnungsgemäßes Auflegen eines Körperteils auf den Auflagebereich anzeigendes Ausgabesignal ausgegeben werden. Ein entsprechendes Ausgabesignal kann beispielsweise auf Grundlage von von wenigstens einer Erfassungseinrichtung gelieferten Erfassungsinformation, d. h. z. B. einer eine erfasste Kontaktierung des Auflagebereichs durch ein Körperteil beschreibenden Erfassungsinformation, erzeugt werden bzw. worden sein. Eine entsprechende Signalausgabeeinrichtung umfasst typischerweise wenigstens ein Signalausgabeelement, welches je nach konkret auszugebendem Signal z. B. akustisches Signalausgabeelement, d. h. z. B. als Lautsprecher, als optisches Signalausgabeelement, d. h. z. B. als Anzeigeelement, Leuchtdiodenelement, etc., oder als haptisches Signalausgabeelement, d. h. z. B. als Vibrationselement, ausgebildet bzw. wenigstens ein solches umfassen kann.

Ein beispielhaft vermittels einer entsprechenden Signalausgabeeinrichtung ausgebbares Ausgabesignal kann wenigstens eine Information betreffend eine nutzerseitige Handlung und/oder eine Bewertung einer nutzerseitigen Handlung, insbesondere an einen Nutzer, der Auflageeinrichtung beinhalten. Derart ist es möglich, vermittels entsprechender entsprechende Informationen beinhaltenden Ausgabesignalen einen Nutzer bei der ordnungsgemäßen Nutzung der Auflageeinrichtung zu instruieren und/oder zu unterstützen. Beispielsweise kann einem Nutzer durch entsprechende Ausgabesignale ausgegeben werden, dass die Auflageeinrichtung zur Auflage eines Körperteils, gegebenenfalls auch welches Körperteils, bereit ist, dass eine ordnungsgemäße Auflage eines Körperteils erfolgt oder (noch) nicht erfolgt ist, wann bzw. dass eine bestimmte, z. B. für eine therapeutische Wirkung zweckmäßige Auflagedauer erreicht oder (noch) nicht erreicht ist, etc. Denkbar sind gleichermaßen einen Nutzer anspornende oder motivierende Ausgabesignale, d. h. Ausgabesignale, welche einen Nutzer, z. B. akustisch, optisch oder haptisch, anspornen bzw. motivieren, die Auflageeinrichtung zu benutzen, eine bestimmte Auflageposition einzunehmen und/oder aufrechtzuerhalten, etc. In analoger Weise sind einen Nutzer bewertende, d. h. insbesondere lobende oder kritisierende, Ausgabesignale denkbar, welche einen Nutzer bzw. eine bestimmte Nutzerhandlung, z. B. akustisch, optisch oder haptisch, vor und/oder während und/oder nach Benutzung der Auflageeinrichtung bewerten.

Der Auflagekörper kann in ein Eingabegerät einer mit einem Eingabegerät steuerbaren Einrichtung, d. h. z. B. eines Endgeräts, wie z. B. eines Computers, eines Multimediageräts, etc. integriert oder als ein solches Eingabegerät ausgebildet sein. Derart kann eine erhöhte Funktionsintegration erzielt werden, welche einem Nutzer zudem die Möglichkeit bietet, auch während der Auflage des Körperteils mit dem Körperteil bestimmte Handlungen, d. h. insbesondere Eingaben, Steuerungen, etc., vorzunehmen. Konkretes Beispiel für ein entsprechendes Eingabegerät ist ein, z. B. als (elektronische) Computermaus ausgebildetes, Eingabeelement zur Tätigung von Eingaben in einen Computer, ein, z. B. als Fernbedienung ausgebildetes, Eingabeelement zur Tätigung von Eingaben in ein Multimediagerät, wie z. B. eine Audioanlage oder einen Fernseher, etc. Weitere Beispiele sind denkbar.

Die Auflageeinrichtung kann eine, insbesondere in den Auflagekörper und/oder in den Auflagebereich integrierte, Temperiereinrichtung zur zumindest abschnittsweisen, gegebenenfalls vollständigen, Temperierung des Auflagekörpers und/oder des Auflagebereichs umfassen. Die vermittels einer entsprechenden Temperiereinrichtung mögliche statische oder dynamische Temperierung des Auflagekörper und/oder des Auflagebereichs - hierunter ist grundsätzlich eine Beheizung als auch eine Kühlung zu verstehen - kann die gewünschte Entspannung eines aufgelegten Körperteils respektive einen durch die Auflage eines Körperteils erzielbaren therapeutischen Effekt unterstützen.

Eine entsprechende Temperiereinrichtung kann eine wenigstens einen von einem Temperiermedium, d. h. z. B. einem Gas oder einer Flüssigkeit, durchströmbaren Temperierkanal umfassende Temperierkanalstruktur umfassen. Eine entsprechende Temperierkanalstruktur kann sich, insbesondere flächig, zumindest abschnittsweise, gegebenenfalls vollständig, in einer oder mehreren Raumrichtungen, d. h. insbesondere in einen oder mehreren horizontalen und/oder vertikalen Raumrichtungen, durch den Auflagekörper bzw. den Auflagebereich erstrecken. Insbesondere kann sich eine entsprechende Temperierkanalstruktur, insbesondere konturnah, im Bereich der bzw. unterhalb der eine tatsächliche Auflagefläche des Auflagekörpers bzw. des Auflagebereichs bildenden (freiliegenden) Oberfläche des Auflagekörpers bzw. des Auflagebereichs erstrecken.

Alternativ oder ergänzend kann eine entsprechende Temperiereinrichtung wenigstens ein elektrisch betreibbares Temperierelement umfassen. Ein entsprechendes elektrisch betreibbares Temperierelement kann z. B. als ein Heizdraht oder eine ein- oder mehrdimensionale Heizdrahtstruktur ausgebildet sein bzw. wenigstens eine(n) solche(n) umfassen. Ein entsprechendes Temperierelement kann sich, insbesondere flächig, zumindest abschnittsweise, gegebenenfalls vollständig, durch den Auflagekörper bzw. den Auflagebereich erstrecken. Insbesondere kann sich ein entsprechendes Temperierelement, insbesondere konturnah, im Bereich der bzw. unterhalb der eine tatsächliche Auflagefläche des Auflagekörpers bzw. des Auflagebereichs bildenden (freiliegenden) Oberfläche des Auflagekörpers bzw. des Auflagebereichs erstrecken.

Alternativ oder ergänzend kann eine entsprechende Temperiereinrichtung wenigstens ein Temperierelement umfassen, welches nach Aktivierung eine, gegebenenfalls reversible, exotherme chemische Reaktion ausführt. Ein entsprechendes Temperierelement kann im Bereich der bzw. unterhalb der eine tatsächliche Auflagefläche des Auflagekörpers bzw. des Auflagebereichs bildenden (freiliegenden) Oberfläche des Auflagekörpers bzw. des Auflagebereichs angeordnet oder ausgebildet sein. Ein entsprechendes Temperierelement kann ein Aktivierungselement, d. h. z. B. eine Lasche, einen Knopf, etc., umfassen, über welches eine Aktivierung des Temperierelements, d. h. eine Initiierung der exothermen Reaktion, möglich ist.

Die Auflageeinrichtung kann ferner eine, insbesondere in den Auflagekörper und/oder in den Auflagebereich integrierte, Belüftungseinrichtung zur zumindest abschnittsweisen, gegebenenfalls vollständigen, Belüftung eines auf der Auflageeinrichtung aufzulegenden oder aufgelegten Körperteils aufweisen bzw. umfassen. Die vermittels einer entsprechenden Belüftungseinrichtung mögliche statische oder dynamische Belüftung des Auflagekörpers und/oder des Auflagebereichs kann die gewünschte Entspannung eines aufgelegten Körperteils respektive einen durch die Auflage eines Körperteils erzielbaren therapeutischen Effekt unterstützen. Ebenso können die Auflageeigenschaften, insbesondere bei Auflegen eines Körperteils über einen längeren Zeitraum, verbessert werden.

Eine entsprechende Belüftungseinrichtung kann eine wenigstens einen von einem Belüftungsmedium, d. h. z. B. einem Gas, durchströmbaren Belüftungskanal umfassende Belüftungskanalstruktur umfassen. Eine entsprechende Belüftungskanalstruktur kann sich, insbesondere flächig, zumindest abschnittsweise, gegebenenfalls vollständig, durch den Auflagekörper bzw. den Auflagebereich erstrecken. Insbesondere kann sich eine entsprechende Belüftungskanalstruktur, insbesondere konturnah, im Bereich der bzw. unterhalb der eine tatsächliche Auflagefläche des Auflagekörpers bzw. des Auflagebereichs bildenden (freiliegenden) Oberfläche des Auflagekörpers bzw. des Auflagebereichs erstrecken.

Eine entsprechende Belüftungskanalstruktur kann durch ein oder mehrere vordefiniert konfigurierte bionische Geometrien ausgebildet sein. Entsprechende bionische Geometrien können eine Belüftung eines auf der Auflageeinrichtung aufzulegenden oder aufgelegten Körperteils unterstützen bzw. verbessern.

Grundsätzlich ist es sonach möglich in dem Auflagekörper, vordefiniert konfigurierte bionische Geometrien zur Ausbildung einer entsprechenden Belüftungskanalstruktur auszubilden.

An dieser Stelle ist anzumerken, dass der Auflagekörper im Allgemeinen zumindest abschnittsweise, gegebenenfalls vollständig, eine vordefiniert konfigurierte bionische Geometrie aufweisen kann.

Die Auflageeinrichtung kann ferner eine dem Auflagekörper und/oder dem Auflagebereich zuordenbare oder zugeordnete Fixiereinrichtung zur zeitweisen Fixierung eines auf den Auflagebereich aufgelegten Körperteils, insbesondere in einer bestimmungsgemäßen Auflageposition des Körperteils auf dem Auflagebereich, aufweisen bzw. umfassen. Eine entsprechende Fixierung kann eine gewünschte Ausrichtung eines auf dem Auflagebereich aufgelegten Körperteils ermöglichen und aufrechterhalten. Derart kann ein vermittels der Auflageeinrichtung realisierbarer Entspannungseffekt bzw. therapeutischer Effekt unterstützt werden. Eine entsprechende Fixiereinrichtung kann eine Fixierung eines jeweiligen Körperteils unmittelbar an dem Auflagekörper respektive dem Auflagebereich ermöglichen; dies kann z. B. über ein an dem Auflagekörper oder dem Auflagebereich anbringbares oder angebrachtes Fixierelement, wie z. B. einen Gurt, eine Schlaufe, etc., realisiert sein, welches das jeweilige Körperteil nach Anlegen zumindest abschnittsweise, gegebenenfalls vollständig, umschließt, sodass das Körperteil an dem Auflagekörper respektive dem Auflagebereich fixiert ist. Denkbar ist auch eine schlauchartige bzw. -förmige Fixiereinrichtung, d. h. z. B. ein z. B. aus einem Textil gebildeter Fixierschlauch, welcher über die Auflageeinrichtung und das auf dieser aufzulegende bzw. aufgelegte Körperteil zu ziehen ist und derart eine gewünschte Ausrichtung eines auf dem Auflagebereich aufgelegten Körperteils ermöglicht und aufrechterhält. Ebenso denkbar ist eine Fixiereinrichtung, welche eine Fixierung eines übergeordneten Körperteils, d. h. z. B. eines Arms, wenn das zu fixierende Körperteil eine Hand ist, in einer bestimmten Ausrichtung ermöglicht.

Die Auflageeinrichtung kann ferner eine Befestigungseinrichtung zur zeitweisen Befestigung der Auflageeinrichtung an oder auf einem Drittgegenstand, insbesondere an oder auf einem an einem Körper eines Nutzers der Auflageeinrichtung zu tragenden Drittgegenstand, aufweisen bzw. umfassen. Die Auflageeinrichtung kann derart auch an Drittgegenständen, d. h. insbesondere mobilen bzw. portablen Drittgegenständen, befestigt werden. Die Befestigung der Auflageeinrichtung an entsprechenden Drittgegenständen kann insbesondere derart sein, dass die Auflageeinrichtung in einer Ausrichtung und/oder Position, in welcher eine gewünschte Ausrichtung eines auf dem Auflagebereich aufgelegten Körperteils möglich ist und aufrechterhalten werden kann. Ein konkretes Beispiel für einen Drittgegenstand ist ein an einem Körper eines Nutzers zu tragender Drittgegenstand, wie z. B. ein Kleidungsstück. Die Auflageeinrichtung kann sonach an oder in einem Drittgegenstand, wie z. B. einem Kleidungsstück, befestigbar sein. Beispielsweise ist es denkbar, dass die Auflageeinrichtung in einer Tasche eines Kleidungsstücks, wie z. B. eines am Oberkörper eines Trägers bzw. einer Trägerin zu tragenden Kleidungsstücks, wie z. B. einer Jacke, eines Pullovers, etc., oder eines am Unterkörper eines Trägers bzw. einer Trägerin zu tragenden Kleidungsstücks, wie z. B. einer Hose, eines Rocks, etc., befestigbar ist. Die Befestigungseinrichtung kann ein oder mehrere Befestigungselemente umfassen, welche zur (beschädigungs- bzw. zerstörungsfreien) Befestigung der Auflageeinrichtung an einem entsprechenden Drittgegenstand eingerichtet sind. Entsprechende Befestigungselemente können z. B. eine form- und/oder kraftschlüssige Befestigung der Auflageeinrichtung an einem entsprechenden Drittgegenstand ermöglichen. Konkretes Beispiel für entsprechende Befestigungselemente sind demnach Haken- und/oder Verschlaufungselemente (Klettverschlusselemente).

Der Auflagekörper kann wenigstens einen Lagerungsabschnitt zur Lagerung des Auflagekörpers auf einem Untergrund bzw. einer Unterlage aufweisen. Über einen entsprechenden Lagerungsabschnitt kann - je nach Ausführung - eine stabile Lagerung des Auflagekörpers auf einem Untergrund bzw. einer Unterlage, wie z. B. einer Tischfläche, realisiert werden.

Ein entsprechender Lagerungsabschnitt kann mit wenigstens einem eine rutschfeste Auflage des Auflagekörpers auf einem Untergrund bzw. einer Unterlage schaffenden Material bzw. einer entsprechenden Materialstruktur versehen sein bzw. ein(e) solche(s) umfassen. Ein entsprechendes Material bzw. eine entsprechende Materialstruktur kann z. B. deshalb zweckmäßig sein, um eine stabile Auflage des Auflagekörpers auf einem Untergrund bzw. einer Unterlage zu ermöglichen. Ein entsprechendes Material bzw. eine entsprechende Materialstruktur kann z. B. aus einem elastomeren Material bzw. einer elastomeren Materialstruktur gebildet sein bzw. wenigstens ein(e) solche(s) umfassen. In Frage kommen grundsätzlich sowohl natürliche als auch synthetische elastomere Materialien bzw. Materialstrukturen. **In** fertigungstechnischer Hinsicht vorteilhaft können z. B. thermoplastische Elastomere verwendet werden.

Ein entsprechender Lagerungsabschnitt kann mit wenigstens einem reversibel verformbaren Material, insbesondere einem reversibel verformbaren weich-elastischen Material, bzw. einer reversibel verformbaren Materialstruktur, insbesondere einer reversibel verformbaren weich-elastischen Materialstruktur versehen sein bzw. ein(e) solche(s) umfassen. Ein entsprechendes Material bzw. eine entsprechende Materialstruktur kann z. B. deshalb zweckmäßig sein, als etwaig erforderliche Änderungen der Ausrichtung des Auflagekörpers auf einem Untergrund bzw. einer Unterlage, insbesondere relativ zu einer Neutral- oder Referenzausrichtung, ermöglichen, welche z. B. Nutzern mit spastischen Körperteilen bzw. Gliedmaßen das Benutzen der Auflageeinrichtung erleichtern bzw. ermöglichen kann; dies gilt insbesondere dann, wenn die jeweilige Spastik oder eine sonstige anatomische Fehlstellung eine besondere Ausrichtung des Auflagekörpers, insbesondere relativ zu einer Neutral- oder Referenzausrichtung, erfordert, um das Körperteil, insbesondere schmerzfrei, auf den Auflagebereich aufzulegen. Ein entsprechendes Material bzw. eine entsprechende Materialstruktur kann z. B. aus einem elastomeren oder zellularen Material bzw. einer elastomeren bzw. zellularen Materialstruktur gebildet sein bzw. wenigstens eine elastomeres Material umfassen. In Frage kommen grundsätzlich sowohl natürliche als auch synthetische elastomere bzw. zellulare Materialien bzw. Materialstrukturen. In fertigungstechnischer Hinsicht vorteilhaft können z. B., gegebenenfalls geschäumte, thermoplastische Elastomere verwendet werden.

Ein entsprechender Lagerungsabschnitt kann wenigstens ein, insbesondere nicht plan auf einem planen Untergrund bzw. einer planen Unterlage aufliegendes, Lagerungsabschnittelement aufweisen, welches eine in wenigstens einem Bewegungsfreiheitsgrad bewegbare, insbesondere schwenkbewegbare, Lagerung des auf dem Untergrund gelagerten Auflagekörpers relativ zu dem Untergrund bzw. der Unterlage ermöglicht. Derart kann der Auflagekörper relativ zu einem Untergrund bzw. einer Unterlage bewegt werden, was vorteilhaft sein kann, um eine möglichst entspannte Positionierung eines jeweiligen Körperteils sowie übergeordneter Körperteile zu realisieren. Beispielsweise kann eine im Vergleich zu einer Referenzausrichtung veränderliche Ausrichtung des Auflagekörpers eine (noch) entspannendere bzw. entspanntere Auflage eines Körperteils ermöglichen. Ein entsprechendes Lagerungsabschnittelement kann z. B. eine ein- oder mehreckige, runde, rundliche oder spitze Geometrie aufweisen. Ebenso kann eine bewegbare Lagerung des Auflagekörpers Trainingsmöglichkeiten, z. B. zum Muskelaufbau, eröffnen, als durch das Bewegen des Auflagekörpers relativ zu dem Untergrund bzw. der Unterlage bestimmte, mit einem Trainingseffekt einhergehende Bewegungsabläufe vollzogen werden können.

Ein entsprechendes Lagerungsabschnittelement kann auch als gesondertes Bauelement ausgebildet sein und mit dem Auflagekörper bedarfsweise unter Ausbildung der Auflageeinrichtung, gegebenenfalls (beschädigungs- bzw. zerstörungsfrei) lösbar, verbindbar oder verbunden sein. Ein entsprechendes, z. B. kugelförmiges, halbkugelförmiges, zylinderförmiges oder halbzylinderförmiges oder pyramidenförmiges, Lagerungsabschnittelement kann z. B. über ein Klett- bzw. Wechselschlusssystem mit dem Auflagekörper verbunden sein.

Die Auflageeinrichtung kann wenigstens ein, insbesondere (beschädigungs- bzw. zerstörungsfrei) lösbar, auf dem Auflagebereich, diesen zumindest abschnittsweise, gegebenenfalls vollständig, abdeckend auflegbares oder aufgelegtes Auflageelement umfassen. Ein entsprechendes Auflageelement kann insbesondere unter hygienischen Gesichtspunkten zweckmäßig sein, als der Auflagebereich respektive der Auflagekörper vor einem direkten Kontakt mit dem jeweiligen Körperteil und somit z. B. vor Ablagerungen der Haut, Schweiß, etc. geschützt ist. Ein entsprechendes Auflageelement kann aus einem elastisch-flexiblen Material bzw. einer elastisch-flexiblen Materialstruktur gebildet sein, sodass das Auflageelement die Passform des Auflagebereichs nicht beeinträchtigt. Ein entsprechendes Auflageelement kann aus einem, gegebenenfalls (ab)waschbaren, natürlichen und/oder synthetischen textilen Material oder einer natürlichen und/oder synthetischen textilen Materialstruktur ausgebildet sein. Lediglich beispielhaft ist auf Leder- oder Kunststoffmaterialien zu verweisen.

Der Auflagekörper und/oder der Auflagebereich können aus wenigstens einem Schaummaterial, d. h. insbesondere aus einem expandierten Partikelschaummaterial, wie z. B. einem expandierten Kunststoffmaterial, gebildet sein oder wenigstens ein solches umfassen. Entsprechende Schaummaterialien reduzieren das Gewicht des Auflagekörpers bzw. des Auflagebereichs. Entsprechende Schaummaterialien können zudem angenehme haptische Eigenschaften des Auflagekörpers bzw. des Auflagebereichs bedingen. Weitere Vorteile entsprechender Schaummaterialien sind deren gut beherrschbare kosteneffiziente Verarbeitung.

Der Auflagekörper und/oder der Auflagebereich können alternativ oder ergänzend aus einem magnetisierbaren oder magnetischen Material gebildet sein oder wenigstens ein solches umfasst. Magnetisierbare oder magnetische Materialien, wie z. B. entsprechende ferromagnetische Metalle, können sich positiv auf den Blutfluss durch ein entsprechendes Körperteil auswirken, was therapeutische Effekte begünstigen kann. Magnetisierbare oder magnetische Materialien können als Vollmaterial oder in Form von Partikeln vorliegen.

Der Auflagekörper und/oder der Auflagebereich können alternativ oder ergänzend aus einem antibakteriellen Material gebildet sein oder wenigstens ein solches umfassen. Antibakterielle Materialien, wie z. B. antibakterielle Metalle, wie z. B. Silber, können sich positiv auf die hygienischen Eigenschaften des Auflagebereichs und/oder des Auflagekörpers auswirken.

Antibakterielle Materialien können als Vollmaterial oder in Form von Partikeln vorliegen.

Im Hinblick auf die Herstellung der Auflageeinrichtung, d. h. des Auflagekörpers und/oder des Auflagebereichs, gilt, dass grundsätzlich sämtliche, insbesondere urformenden und/oder formgebenden, Fertigungsverfahren in Betracht kommen.

Insbesondere kommen sowohl additive (aufbauende) Fertigungsverfahren, auch subtraktive (abtragende) Fertigungsverfahren, d. h. insbesondere spanabhebende Fertigungsverfahren, als auch formgebende Fertigungsverfahren, wie z. B. Tiefziehen, in Betracht.

Additive Fertigungsverfahren können zweckmäßig sein, als diese geeignet sind, entsprechende vordefiniert bzw. standardisiert konfigurierte geometrisch-konstruktive Gestaltungen auszubilden. Demnach können additive Fertigungsverfahren, welche einen additiven Aufbau dreidimensionaler Objekte durch sukzessive schichtweise selektive Verfestigung eines sukzessive schichtweise selektiv verfestigbaren, gegebenenfalls pulverförmigen, Baumaterials - hierbei kann es sich z. B. um ein Metall oder um einen Kunststoff handeln - ermöglichen, in Betracht. Lediglich beispielhaft sei in diesem Zusammenhang auf Binder-Jet-Verfahren, selektive Laserschmelzverfahren, selektive Lasersinterverfahren oder Stereolithographieverfahren, etc. verwiesen.

An dieser Stelle ist zu erwähnen, dass die Auflageeinrichtung grundsätzlich aus jedwedem Material gefertigt werden kann. Lediglich beispielhaft wird auf Kunststoffe, Metalle, Keramiken verwiesen. Auch eine Ausbildung aus einem mehrere unterschiedliche, d. h. sich in wenigstens einem chemischen und/oder physikalischen Parameter unterscheidende, Materialien bzw. Materialstrukturen aufweisenden Verbundmaterial ist denkbar.

Ein zweiter Aspekt der Erfindung betrifft ein Kommunikationssystem, welches wenigstens eine wie hierin beschriebene Auflageeinrichtung, welche eine Übertragungseinrichtung, welche zur Übertragung einer Information an wenigstens einen Kommunikationspartner zum Empfang einer von wenigstens einem Kommunikationspartner übertragenen Information eingerichtet ist, sowie wenigstens einen Kommunikationspartner, welcher zum Empfang einer von der wenigstens einen Auflageeinrichtung übertragenen Information und/oder zur Übertragung einer Information an die wenigstens eine Auflageeinrichtung eingerichtet ist, umfasst.

Sämtliche Ausführungen im Zusammenhang mit der Auflageeinrichtung, insbesondere sämtliche Ausführungen im Zusammenhang mit etwaigen Übertragungseinrichtungen der Auflageeinrichtung, gelten analog für das Kommunikationssystem.

Ein dritter Aspekt der Erfindung betrifft ein Verfahren zur Herstellung einer Aufnahmeeinrichtung zur zumindest abschnittsweisen, gegebenenfalls vollständigen, Auflage wenigstens eines Körperteils eines Nutzers, welches Verfahren wenigstens die Schritte umfasst:
- Bereitstellen einer die Abmessungen eines auf einer herzustellenden Auflageeinrichtung aufzulegenden Körperteils zumindest abschnittsweise, gegebenenfalls vollständig, beschreibenden Körperteilabmessungsinformation;
- Bereitstellen, z. B. in einer Datenspeichereinrichtung, mehrerer Auflageeinrichtungsabmessungsinformationen, wobei jede Auflageeinrichtungsabmessungsinformation wenigstens eine wenigstens einer Abmessung eines Körperteils zugeordnete vordefinierte Abmessung einer auf Grundlage der jeweiligen Auflageeinrichtungsabmessungsinformation herstellbaren bzw. herzustellenden Auflageeinrichtung beschreibt;
- Auswählen einer Auflageeinrichtungsabmessungsinformation auf Grundlage der bereitgestellten Körperteilabmessungsinformation;
- Herstellen einer Auflageeinrichtung auf Grundlage von die ausgewählte Auflageeinrichtungsabmessungsinformation beinhaltenden oder beschreibenden Daten.

Entsprechende Daten, auf Grundlage welche die Auflageeinrichtung verfahrensgemäß hergestellt wird, können sonach Herstellungsdaten einer zur Herstellung der jeweiligen Auflageeinrichtung verwendbaren bzw. verwendeten Herstellungsanlage beinhalten oder in solche Herstellungsdaten umgewandelt werden.

Sämtliche Ausführungen im Zusammenhang mit der Auflageeinrichtung, insbesondere sämtliche Ausführungen im Zusammenhang mit der Herstellung der Auflageeinrichtung, gelten analog für das Verfahren.

Das Auswählen kann verfahrensgemäß durch Abgleich oder Vergleich der bereitgestellten Körperteilabmessungsinformation mit jeweiligen jeweiligen Auflageeinrichtungsabmessungsinformationen zugeordneten Körperteilabmessungsinformationen erfolgen, wobei eine Auflageeinrichtungsabmessungsinformation ausgewählt werden kann, deren zugeordnete Körperteilabmessungsinformation der bereitgestellten Körperteilabmessungsinformation gleicht oder, insbesondere im Rahmen eines vordefinierbaren oder vordefinierten Toleranzbereichs, ähnelt.

Ein entsprechender Abgleich kann z. B. durch einen Abgleich wenigstens einer in der oder einer bereitgestellten Körperteilabmessungsinformation beschriebenen Abmessung, wie z. B. Länge, Breite, Höhe, etc., eines Körperteils oder eines Körperteilabschnitts oder eines Abmessungsverhältnisses, wie z. B. eines Längen-Breiten-Verhältnisses, eines Körperteils oder eines Körperteilabschnitts beschreiben, mit wenigstens in der oder einer Auflageeinrichtungsabmessungsinformationen beschriebenen korrespondierenden Abmessungen, d. h. z. B. Länge, Breite, Höhe, etc., oder eines korrespondierenden Abmessungsverhältnisses, wie z. B. eines Längen-Breiten-Verhältnisses, erfolgen. Ein entsprechender Abgleich kann durch einen Benutzer, d. h. z. B. einen Träger der herzustellenden Auflageeinrichtung oder einen Hersteller der herzustellenden Auflageeinrichtung, erfolgen. Alternativ oder ergänzend kann ein entsprechender Abgleich, gegebenenfalls automatisierbar oder automatisiert, durch eine hardware- und/oder softwaremäßig implementierte und entsprechend hardware- oder softwaremäßig zur Durchführung eines entsprechenden Abgleichs konfigurierte Abgleicheinrichtung erfolgen. Eine entsprechende Abgleicheinrichtung kann einen hardware- und/oder softwaremäßigen Bestandteil einer übergeordneten mobilen oder stationären Datenverarbeitungseinrichtung bilden, welche sonach zur Durchführung eines entsprechenden Abgleichs konfiguriert sein kann.

Ein entsprechender Vergleich kann durch einen Vergleich wenigstens einer in der oder einer bereitgestellten Körperteilabmessungsinformation beschriebenen Abmessung, wie z. B. Länge, Breite, Höhe, etc., eines Körperteils oder eines Körperteilabschnitts oder eines Abmessungsverhältnisses, wie z. B. eines Längen-Breiten-Verhältnisses, eines Körperteils oder eines Körperteilabschnitts beschreiben, mit wenigstens in der oder einer Auflageeinrichtungsabmessungsinformationen beschriebenen korrespondierenden Abmessungen, d. h. z. B. Länge, Breite, Höhe, etc., oder eines korrespondierenden Abmessungsverhältnisses, wie z. B. eines Längen-Breiten-Verhältnisses, erfolgen.

Eine verfahrensgemäß bereitzustellende bzw. bereitgestellte Körperteilabmessungsinformation kann durch, insbesondere optisches, Erfassen eines auf der Auflageeinrichtung aufzulegenden Körperteils eines Nutzers erfolgen. Verfahrensgemäß kann sonach ein, insbesondere optisches, Erfassen eines auf der Auflageeinrichtung aufzulegenden Körperteils eines Nutzers und ein Erzeugen einer bereitzustellenden Körperteilabmessungsinformation auf Grundlage einer durch das Erfassen erzeugten Erfassungsinformation erfolgen. Das Erfassen kann, wie angedeutet, insbesondere optisch erfolgen, sodass ein Erfassen z. B. vermittels einer Erfassungseinrichtung in Form einer Kamera- oder Videoeinrichtung durchgeführt werden kann. Eine entsprechende Kamera- oder Videoeinrichtung kann Bestandteil eines nutzer- oder herstellerseitigen, insbesondere mobilen, Endgeräts, wie z. B eines mobilen Computers (Laptop), eines Smartphones, eines Tablets, einer Smartbrille, etc., bilden.

Im Allgemeinen kann das Erfassen eines entsprechenden Körperteils vermittels einer Rohdatenerfassungseinrichtung erfolgen, welche z. B. als eine, insbesondere mechanische oder optische, Erfassungseinrichtung, welche zum Erfassen ein- oder mehrdimensionaler, die Abmessungen und/oder die Morphologie wenigstens eines Körperteils beschreibenden Körperteilrohdaten eingerichtet ist. Eine mechanische Erfassungseinrichtung kann z. B. als Messeinrichtung ausgebildet sein, welche ein Erfassen von die Morphologie wenigstens eines Körperteils beschreibenden Körperteilrohdaten auf Grundlage von erfassten mechanischen Größen, d. h. insbesondere Druck, ermöglicht. Eine mechanische Erfassungseinrichtung kann insbesondere auch zur Erstellung eines Körperteilabdrucks eingerichtet sein. Eine optische Erfassungseinrichtung kann, wie erwähnt, z. B. als Kamera-, Video- oder als Scaneinrichtung, insbesondere als Körperteilscaneinrichtung, ausgebildet sein. In allen Fällen ist es auch denkbar, dass eine entsprechende Rohdatenerfassungseinrichtung als eine Erfassungseinrichtung zum, insbesondere mechanischen und/oder optischen, Erfassen ein- oder mehrdimensionaler Abmessungen, d. h. z. B. Länge, Breite, Höhe, Umfang, Volumen, eines Körperteils ausgebildet ist oder wenigstens eine solche umfasst.

Die Erfindung ist anhand von Ausführungsbeispielen in den Zeichnungen näher erläutert. Dabei zeigt:
Fig. 1 - 16 jeweils eine Prinzipdarstellung einer Auflageeinrichtung zur Auflage eines Körperteils eines Nutzers gemäß einem Ausführungsbeispiel;
Fig. 17 eine Prinzipdarstellung eines Kommunikationssystems gemäß einem Ausführungsbeispiel; und
Fig. 18 eine Prinzipdarstellung eines Verfahrens zur Herstellung einer Auflageeinrichtung zur Aufnahme eines Körperteils eines Nutzers gemäß einem Ausführungsbeispiel.

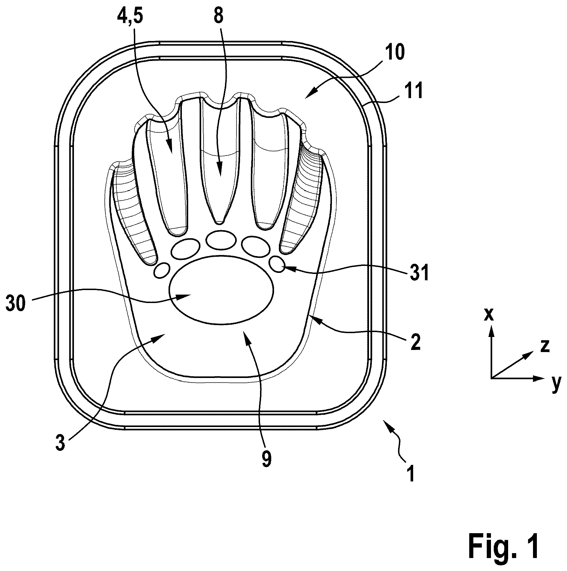

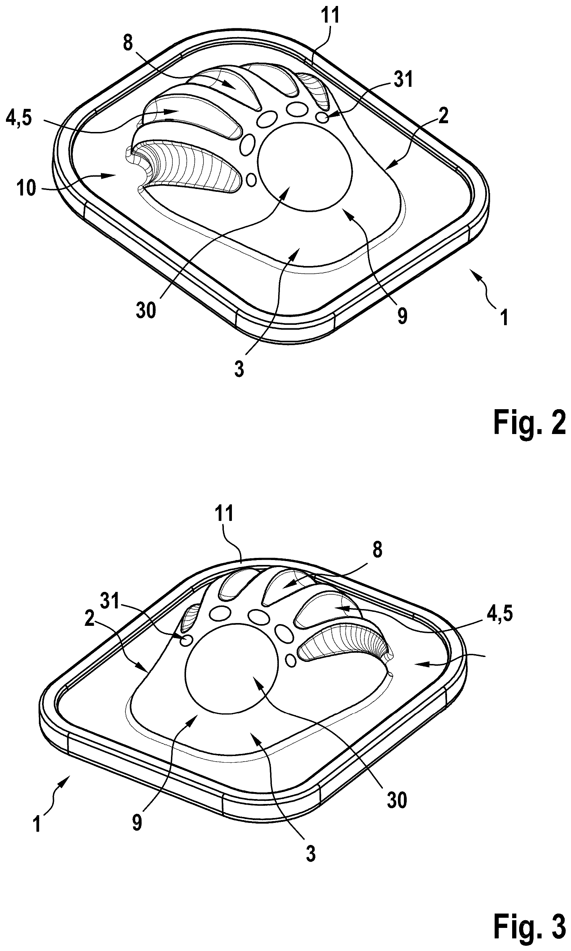

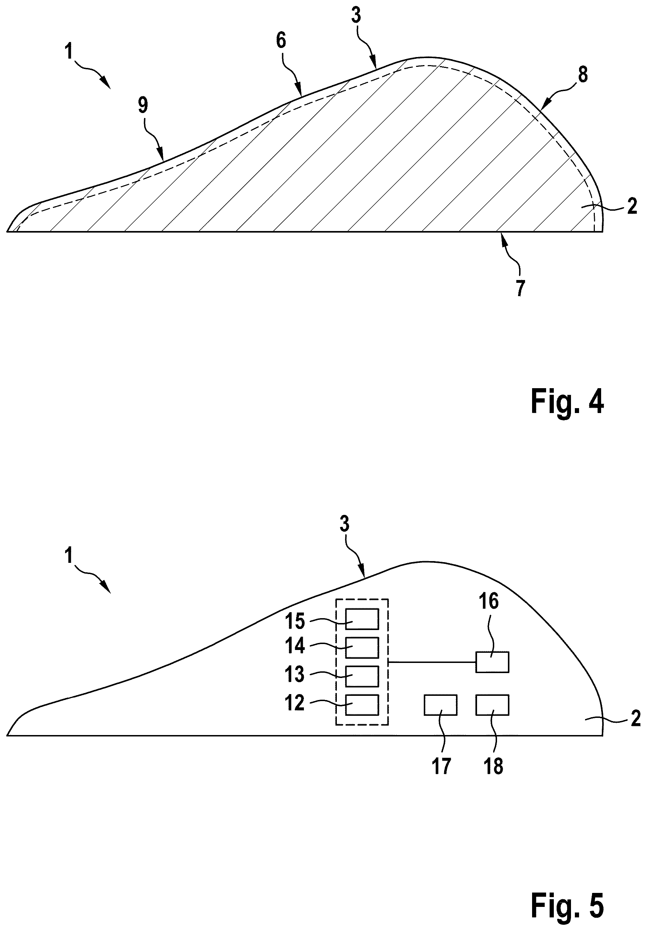

Die Fig. 1 - 4 zeigen Fig. eine Prinzipdarstellung einer Auflageeinrichtung 1 zur Auflage eines Körperteils (nicht gezeigt) eines Nutzers gemäß einem Ausführungsbeispiel in einer Aufsicht (Fig. 1), in einer ersten perspektivischen Ansicht aus einer ersten Blickrichtung (Fig. 2), in einer zweiten perspektivischen Ansicht aus einer zweiten Blickrichtung (Fig. 3) und in einer geschnittenen Seitenansicht (Fig. 4).

Die Auflageeinrichtung 1 dient im Allgemeinen zur Auflage bzw. Lagerung wenigstens eines Körperteils, d. h. in den in den Fig. gezeigten Ausführungsbeispielen einer Hand, eines Nutzers bzw. einer Nutzerin.

Die Auflageeinrichtung 1 umfasst einen Auflagekörper 2. Der auch als Lagerungskörper zu bezeichnende bzw. zu erachtende Auflagekörper 2 weist einen auch als Lagerungsbereich zu bezeichnenden bzw. zu erachtenden Auflagebereich 3 zur Auflage eines Körperteils eines Nutzers auf. Der Auflagebereich 3 ist sonach als derjenige Bereich der Auflageeinrichtung 1 zu verstehen, auf welchem ein Nutzer bei bestimmungsgemäßer Nutzung der Auflageeinrichtung 1 ein Körperteil (direkt) auflegt. Der Auflagebereich 3 stellt sonach den eigentlichen Bereich der Auflageeinrichtung 1, auf welchem ein Nutzer bei bestimmungsgemäßer Nutzung der Auflageeinrichtung 1 ein Körperteil auflegt bzw. lagert, dar. Der Auflagebereich 3 bildet, wie Fig. 1 zeigt, typischerweise einen Teil der (freiliegenden) Oberfläche der Auflageeinrichtung 1.

Die Auflageeinrichtung 1 umfasst einen vordefiniert konfiguriert ausgebildeten Auflagekörper 2, welcher wenigstens eine Oberfläche aufweist. Die Oberfläche des Auflagekörpers 2 weist eine im Hinblick auf ein bestimmtes Körperteil eines bestimmten Nutzers vordefiniert konfiguriert ausgebildete dreidimensionale Konturierung bzw. Strukturierung auf, welche insbesondere durch den Auflagebereich 3 gebildet ist. Der Auflagebereich 3 kann dabei einstückig mit dem Auflagekörper 2 ausgebildet sein oder als ein gesondertes Bauteil ausgebildet sein, welches mit dem Auflagekörper 2 unter Ausbildung der Auflageeinrichtung 1 verbindbar oder verbunden ist.

Die Abmessungen des Auflagekörpers 2 respektive des Auflagebereichs 3 sind typischerweise an die Abmessungen des jeweils aufzulegenden Körperteils angepasst. Bei bestimmungsgemäßer Nutzung der Auflageeinrichtung 1 ist der Auflagebereich 3 durch den jeweils aufgelegten Körperteil sonach zumindest abschnittsweise, gegebenenfalls vollständig, abgedeckt.

Zumindest der Auflagebereich 3 weist eine auf Grundlage einer Körperteilabmessungsinformation, welche die Abmessungen eines auf die Auflageeinrichtung 1 aufzulegenden Körperteils beschreibt, aus einer Mehrzahl an vordefinierten Auflageeinrichtungsabmessungsinformationen, wobei jede Auflageeinrichtungsabmessungsinformation wenigstens einer Abmessung eines Körperteils zugeordnete vordefinierte Abmessungen einer auf Grundlage der jeweiligen Auflageeinrichtungsabmessungsinformation herstellbaren Auflageeinrichtung 1 beschreibt, ausgewählten Auflageeinrichtungsabmessungsinformation beschreibenden Daten vordefiniert konfiguriert ausgebildete geometrisch-konstruktive Gestaltung bzw. Formgebung auf. Der Auflagebereich 3 weist sonach eine vordefiniert konfigurierte geometrisch-konstruktive Gestaltung bzw. Formgebung auf, welche auf Grundlage von durch eine Auflageeinrichtungsabmessungsinformation beschriebenen Daten ausgebildet bzw. erzeugt ist.

Die die Grundlage der für die Ausbildung bzw. Erzeugung des Auflagebereichs verwendeten Daten bildende Auflageeinrichtungsabmessungsinformation ist aus einer Mehrzahl an vordefinierten Auflageeinrichtungsabmessungsinformationen, wobei jede Auflageeinrichtungsabmessungsinformation wenigstens einer Abmessung, wie z. B. Länge, Breite, Höhe, etc., oder wenigstens eines Abmessungsverhältnisses, wie z. B. eines Länge-Breite-Verhältnisses, eines Länge-Höhe-Verhältnisses, eines Breite-Höhe-Verhältnisses, eines Länge-Breite-Höhe-Verhältnisses, etc., eines Körperteils zugeordnete vordefinierte Abmessungen der auf Grundlage der jeweiligen Auflageeinrichtungsabmessungsinformation herstellbaren bzw. hergestellten Auflageeinrichtung 1 beschreibt, ausgewählt. Die Auswahl der Auflageeinrichtungsabmessungsinformation aus der Mehrzahl an Auflageeinrichtungsabmessungsinformationen ist auf Grundlage einer Körperteilabmessungsinformation, welche die Abmessungen eines auf die Auflageeinrichtung 1 aufzulegenden Körperteils zumindest abschnittsweise, gegebenenfalls vollständig, beschreibt, erfolgt.

Die vordefiniert konfigurierte geometrisch-konstruktive Gestaltung bzw. Formgebung des Auflagebereichs 3 eröffnet erhebliche fertigungstechnische Vorteile, als es sich bei dem Auflagebereich 3 nicht um einen individuell konfiguriert ausgebildeten Bauteilabschnitt bzw. nicht um ein individuell konfiguriert ausgebildetes Bauteil handelt. Der Auflagebereich 3 ist nämlich vordefiniert konfiguriert, d. h. er weist eine konkrete vordefiniert konfigurierte räumlich-körperliche Gestaltung und somit eine konkrete vordefiniert konfigurierte Raumform auf, welche, wenngleich diese in abstrahierter bzw. standardisierter Form, im Hinblick auf gewisse Merkmale eines jeweilig aufzulegenden Körperteils, d. h. in den in den Fig. gezeigten Ausführungsbeispielen Anzahl und Anordnung von Fingern einer (menschlichen) Hand, ausgebildete Auflageabschnitte 4 aufweist, nicht im Hinblick auf ein auf der Auflageeinrichtung 1 aufzulegendes Körperteil individualisiert ist.

Die Auflageeinrichtung 1 weist sonach keine individuell konfiguriert ausgebildete geometrisch-konstruktive Gestaltung und somit, abgesehen von einer etwaigen Anpassung bzw. Skalierung der Abmessungen, keine Individualisierung auf. Bei. der Auflageeinrichtung 1 bzw. dem Auflagebereich 3 handelt es sonach nicht um ein für ein bestimmtes Körperteil eines bestimmten Nutzers individuell konfiguriertes Einzelteil. Vielmehr ist die Auflageeinrichtung 1 bzw. der Auflagebereich 3 als ein im Hinblick auf bestimmte Abmessungen eines Körperteils, jedoch im Übrigen völlig unabhängig von individuellen Merkmalen des jeweiligen Körperteils, als ein fertigungstechnisch einfach herstellbares Massenbauteil. Die Auflageeinrichtung 1 bzw. der Auflagebereich 3 weist aufgrund der vordefiniert konfigurierten geometrisch-konstruktiven Gestaltung bzw. Formgebung eine standardisierte geometrisch-konstruktive Gestaltung bzw. Formgebung auf, welche, wenn überhaupt, allein durch Skalierung im Hinblick auf die Abmessungen eines konkret aufzulegenden Körperteils angepasst ist bzw. wird.

Ersichtlich stellt die vordefiniert konfigurierte geometrisch-konstruktive Gestaltung des Auflagebereichs 3 - hierunter ist insbesondere eine vordefiniert konfigurierte dreidimensionale Konturierung bzw. Strukturierung der Oberfläche des Auflagebereichs 3 zu verstehen - ein vordefiniert abstrahiert bzw. standardisiert konfiguriertes (negatives) Abbild eines auf dem Auflagebereich 3 aufzulegenden Körperteils dar. Die Abstrahierung bzw. Standardisierung resultiert - trotz dessen es sich nicht um eine nutzerspezifisch bzw. körperteilspezifisch individuell konfigurierte Passform der Auflageeinrichtung 1 handelt - in einer ausreichend genauen Passform des Auflagebereichs 3, welche eine Auflage eines jeweiligen Körperteils auf dem Auflagebereich 3 in einer körperteilspezifisch entspannenden bzw. entspannten Position ermöglicht.

Eine Auflage eines Körperteils in einer körperteilspezifisch entspannenden bzw. entspannten Position kann sowohl einen Entspannungseffekt auf das jeweilige Körperteil als auch auf an das jeweilige Körperteil anschließende weitere Körperteile bewirken, sodass ein Nutzer bei Benutzung der Auflageeinrichtung 1 insgesamt in einen entspannenden bzw. entspannten Zustand gebracht werden kann. Neben dem reinen Entspannungseffekt respektive damit einhergehend können, soweit erforderlich, auch Therapieeffekte bewirkt werden, als durch die Auflage des Körperteils in einer körperteilspezifisch entspannenden bzw. entspannten Position z. B. die Durchblutung und/oder die Sauerstoffversorgung des jeweiligen Körperteils therapeutisch wirksam beeinflusst werden kann. Positive Effekte können insbesondere bei rheumatischen Körperteilen bzw. Gliedmaßen und/oder bei, z. B. aufgrund eines Schlaganfalls, spastischen Körperteilen bzw. Gliedmaßen erreicht werden. Die mit der Spastik einhergehende Erhöhung der Eigenspannung (Tonus) der Muskulatur des jeweiligen Körperteils kann durch (regelmäßige) Benutzung der Auflageeinrichtung (teilweise deutlich) reduziert werden.

Die Auflageeinrichtung 1 kann dabei ohne Hilfestellung durch einen Dritten benutzt werden; mithin kann der Nutzer im Sinne einer Selbsthilfe durch bestimmungsgemäße Benutzung der Auflageeinrichtung 1 selbstständig therapeutische Maßnahmen, z. B. zur Tonusregulation, Schmerztherapie, etc., vornehmen bzw. sich auf entsprechende therapeutische Maßnahmen vorbereiten.

Für das Beispiel einer spastischen Hand ermöglicht es die insoweit auch als Handballenauflage für die Therapie von Personen mit einer krampfenden oder verkrampften Hand zu bezeichnende bzw. zu erachtende Auflageeinrichtung 1, dass die Hand auf der Auflageeinrichtung 1 in einem entkrampften Zustand aufliegt, wodurch ein Entspannungszustand erreicht wird.

Anhand von Fig. 1 ist ersichtlich, dass der Auflagebereich 3 integral bzw. einstückig mit dem Auflagekörper 2 ausgebildet sein kann. Der Auflagebereich 3 bildet hier eine (freiliegende) Oberfläche des Auflagekörpers 2. Eine (freiliegende) Oberfläche des Auflagekörpers 2 ist sonach mit einer individuell konfiguriert ausgebildeten geometrisch-konstruktiven Gestaltung ausgeführt.

Anhand von Fig. 4 ist, wie durch die strichlierte Darstellung rein beispielhaft angedeutet, ersichtlich, dass der Auflagebereich 3 als zu dem Auflagekörper 2 gesondertes Bauelement ausgebildet und mit dem Auflagekörper 2 bedarfsweise unter Ausbildung der Auflageeinrichtung 1, gegebenenfalls (beschädigungs- bzw. zerstörungsfrei) lösbar, verbindbar oder verbunden sein kann. In dieser Variante kann der Auflagekörper 2 und/oder der Auflagebereich 3 geeignete, gegebenenfalls korrespondierende, Verbindungselemente (nicht gezeigt) umfassen, welche eine stabile, gleichwohl typischerweise (beschädigungs- bzw. zerstörungsfrei) lösbare, Verbindung des Auflagekörpers 2 mit dem Auflagebereich 3, und umgekehrt, ermöglichen. Der Auflagekörper 2 kann z. B. wenigstens ein erstes Verbindungselement und der Auflagebereich 3 wenigstens ein zweites Verbindungselement aufweisen, wobei das wenigstens eine erste und das wenigstens eine zweite Verbindungselement eingerichtet sind, miteinander unter Ausbildung der Auflageeinrichtung 1 zusammenzuwirken. Das wenigstens eine erste Verbindungselement und das wenigstens eine zweite Verbindungselement können z. B. eingerichtet sein, miteinander unter Ausbildung der Auflageeinrichtung 1 form- und/oder kraftschlüssig zusammenzuwirken. Denkbar ist es auch, dass das wenigstens eine erste Verbindungselement und das wenigstens eine zweite Verbindungselement eingerichtet sind, miteinander unter Ausbildung der Auflageeinrichtung 1 magnetisch zusammenzuwirken.

Anhand der Fig. 1 - 4 ist ersichtlich, dass der Auflagebereich 3 mehrere vordefiniert konfiguriert ausgebildete Auflageabschnitte 4, welche zur Aufnahme eines bestimmten Körperteilabschnitts bzw. eines Gliedmaßenabschnitts, eines auf dem Auflagebereich 3 aufzulegenden bzw. aufgelegten Körperteils bzw. einer auf dem Auflagebereich 3 aufzulegenden bzw. aufgelegten Gliedmaße, eingerichtet sind, aufweist. Die für die Auflage eines bestimmten Körperteils vorgesehene Auflageeinrichtung 1 kann sonach eine Vielzahl an körperteilspezifisch vordefiniert konfiguriert ausgebildeten Auflageabschnitten 4 aufweisen. Die Auflageabschnitte 4 sind in den in den Fig. gezeigten Ausführungsbeispielen als vordefiniert konfigurierte Aufnahmen 5 ausgebildet.

In den in den Fig. gezeigten Ausführungsbeispielen weist der Auflagebereich 3 eine bestimmte Anzahl und Anordnung an vordefiniert konfiguriert ausgebildeten Auflageabschnitten 4 auf, welche der Anzahl und Anordnung der der Finger einer jeweiligen auf der Auflageeinrichtung 1 aufzulegenden Hand eines Nutzers entsprechen. Die Auflageabschnitte 4 können sonach als Fingerauflageabschnitte bezeichnet bzw. erachtet werden.

Konkret weist der Auflagebereich in den in den Fig. gezeigten Ausführungsbeispielen fünf vordefiniert konfiguriert ausgebildete Fingerauflageabschnitte in (im Wesentlichen) paralleler Anordnung auf, welche (im Wesentlichen) der Anzahl und Anordnung der Finger einer jeweiligen auf der Auflageeinrichtung 1 aufzulegenden Hand eines Nutzers entspricht. Jeweilige Fingerauflageabschnitte sind ebenso vordefiniert konfiguriert bzw. standardisiert ausgebildet. Konkret sind jeweilige Fingerauflageabschnitte in den in den Fig. gezeigten Ausführungsbeispielen beispielhaft als vordefiniert bzw. standardisiert konfiguriert ausgebildete Aufnahmen bzw. Vertiefungen ausgebildet.

Ersichtlich sind die Fingerauflageabschnitte sich in einer ersten Raumrichtung (vgl. x-Achse in Fig. 1), insbesondere einer distalen Raumrichtung bezogen auf eine auf der Auflageeinrichtung 1 aufgelegte Hand, ersteckend angeordnet und/oder ausgerichtet.

Anhand der Fig. 1 - 3 ist ferner ersichtlich, dass die Auflageeinrichtung 1 ein oder mehrere, insbesondere sich quer zu den Fingerauflageabschnitten erstreckend angeordnete oder ausgerichtete, weitere Auflageabschnitte 31 zur Aufnahme eines Endbereichs eines (proximalen) Mittelhandbereichs einer auf der Auflageeinrichtung 1 aufzulegenden Hand eines Nutzers aufweisen bzw. umfassen kann. Diese weiteren Auflageabschnitte 31 sind ebenso vordefiniert konfiguriert bzw. standardisiert ausgebildet und können konkret z. B. als vordefiniert bzw. standardisiert konfiguriert ausgebildete Aufnahmen bzw. Vertiefungen ausgebildet sein.

Jeweilige Fingerauflageabschnitte können, insbesondere unmittelbar, in einen entsprechenden weiteren Auflageabschnitt übergehen, d. h., insbesondere unmittelbar, mit einem entsprechenden weiteren Auflageabschnitt kommunizieren.

Anhand der Fig. 1 - 4 ist ersichtlich, dass der Auflagekörper 2 bzw. der Auflagebereich 3 einen Grundabschnitt 6 aufweisen bzw. umfassen, welcher mehrere relativ zu einer ungewölbten

Grundebene 7, insbesondere einer Hand nachempfunden, gewölbt ausgebildete Wölbungsabschnitte 8, 9 aufweist. Die Wölbungsabschnitte 8, 9 sind ebenso vordefiniert konfiguriert bzw. standardisiert ausgebildet und können, insbesondere im Bereich der Mittelhand, z. B. als vordefiniert bzw. standardisiert konfiguriert ausgebildete, insbesondere pelottenartige bzw. -förmige, Erhöhungen ausgebildet sein.

Anhand der Fig. 1 - 3 ist ferner ersichtlich, dass der bei bestimmungsgemäßer Nutzung der Auflageeinrichtung 1 proximale Grundabschnitt 9 wenigstens einen pelottenartigen bzw. - förmigen Wölbungsabschnitt 30 aufweisen kann, welcher zur Stützung eines Mittelhandbereichs einer auf der Auflageeinrichtung 1 aufgelegten Hand eines Nutzers eingerichtet ist.

Die Fingerauflageabschnitte sind in den in den Fig. gezeigten Ausführungsbeispielen in dem bei bestimmungsgemäßer Nutzung der Auflageeinrichtung 1 distalen ringsegmentartig oder -förmig gewölbt ausgebildeten Wölbungsabschnitt 8 ausgebildet.

Ganz allgemein weist der Auflagekörper 2 bzw. der Auflagebereich 3 eine einer auf der Auflageeinrichtung 1 aufzulegenden Hand nachempfunden vordefiniert konfiguriert ausgebildete Grundform auf.

In den in den Fig. gezeigten Ausführungsbeispielen ist der Auflagekörper 2 auf einer Grund- oder Trägerplatte 10 (optional) angeordnet oder ausgebildet. Die Grund- bzw. Trägerplatte 10 kann mit wenigstens einer haftungssteigernden Strukturierung, wie z. B. einer besonderen Oberflächenstrukturierung, z. B. einer Aufrauung, einer Gummierung, etc., versehen sein, um eine stabile Auflage bzw. Lagerung der Auflageeinrichtung 1 auf einem Untergrund sicherzustellen.

Die, wie angedeutet, grundsätzlich optionale, Grund- bzw. Trägerplatte 10 kann einen Bestandteil einer Trägereinrichtung (nicht näher bezeichnet) zum Tragen der Auflageeinrichtung 1 bilden. Die Grund- bzw. Trägerplatte 10 kann sonach nebst darauf angeordnetem oder ausgebildetem Auflagekörper 2, z. B. durch Auf- oder Einsetzen, mit einem, z. B. rahmenartigen bzw. -förmigen, Trägerelement 11 bedarfsweise unter Ausbildung einer entsprechenden Trägereinrichtung, gegebenenfalls (beschädigungs- bzw. zerstörungsfrei) lösbar, verbindbar oder verbunden sein. Die Grund- bzw. Trägerplatte 10 bzw. das Trägerelement 11 können geeignete, gegebenenfalls korrespondierende, Verbindungselemente umfassen, welche eine stabile, gleichwohl typischerweise (beschädigungs- bzw. zerstörungsfrei) lösbare, Verbindung der Grund- bzw. Trägerplatte 10 mit dem Trägerelement 11, und umgekehrt, ermöglichen.

Fig. 5 zeigt eine Prinzipdarstellung einer Auflageeinrichtung 1 zur Auflage eines Körperteils (nicht gezeigt) eines Nutzers gemäß einem weiteren Ausführungsbeispiel in einer Seitenansicht.

Anhand des in Fig. 5 gezeigten Ausführungsbeispiels ist ersichtlich, dass die Auflageeinrichtung 1 eine, insbesondere in den Auflagekörper 2 integrierte, Erfassungseinrichtung 12 umfassen kann, welche zur Erfassung wenigstens einer chemischen und/oder physikalischen Größe des Auflagebereichs 3 und/oder des Auflagekörpers 2 und/oder eines auf dem Auflagebereich 3 aufgelegten Körperteils eingerichtet ist. Eine entsprechende Erfassungseinrichtung 12 kann z. B. zur Erfassung chemischer Parameter, wie z. B. der Zusammensetzung der Oberfläche eines aufgelegten Körperteils oder einer Substanz auf der Oberfläche eines aufgelegten Körperteils, und/oder thermischer Parameter, wie z. B. der Temperatur des Auflagekörpers 2 und/oder eines aufgelegten Körperteils, und/oder mechanischer Parameter, wie z. B. von, insbesondere vermittels eines aufgelegten Körperteils, auf den Auflagekörper 2 und/oder den Auflagebereich 3 ausgeübten, Kräften, d. h. insbesondere Gewichtskräften, Druck, Spannung, etc., und/oder hydrometrischer Parameter, wie z. B. der Feuchtigkeit eines aufgelegten Körperteils, etc. eingerichtet sein. Eine entsprechende Erfassungseinrichtung 12 kann ein oder mehrere, z. B. als Sensoren ausgebildete oder solche umfassende, Erfassungselemente (nicht gezeigt) umfassen, welche an oder in dem Auflagekörper 2 und/oder an oder in dem Auflagebereich 3 angeordnet oder ausgebildet sind. Eine entsprechende Erfassungseinrichtung 12 verfügt typischerweise über geeignete hardware- und/oder softwaremäßig implementierte Auswertungs- und/oder Verarbeitungsvorkehrungen, d. h. z. B. über geeignete Auswertungs- und/oder Verarbeitungsalgorithmen, welche eine datenmäßige Auswertung bzw. Verarbeitung jeweiliger erfassungselementseitig gelieferter Erfassungssignale ermöglichen.

Anhand von Fig. 5 ist ferner ersichtlich, dass die Auflageeinrichtung 1 eine, insbesondere in den Auflagekörper 2 integrierte, Erfassungseinrichtung 13 umfassen kann, welche zur Erfassung eines auf den Auflagebereich 3 aufgelegten Körperteils eingerichtet ist. Eine entsprechende Erfassungseinrichtung 13 kann z. B. zur Erfassung einer Annäherung bzw. eines Kontakts eines auf den Auflagebereich 3 aufzulegenden bzw. aufgelegten Körperteils an den bzw. mit dem Auflagebereich 3 eingerichtet sein. Eine entsprechende Erfassungseinrichtung 13 kann ein oder mehrere, z. B. als Sensoren, insbesondere Näherungs- bzw. Kontaktsensoren, ausgebildete oder solche umfassende, Erfassungselemente (nicht gezeigt) umfassen, welche an oder in dem Auflagekörper 2 und/oder an oder in dem Auflagebereich 3 angeordnet oder ausgebildet sind. Eine entsprechende Erfassungseinrichtung 13 verfügt typischerweise über geeignete hardware- und/oder softwaremäßig implementierte Auswertungs- und/oder Verarbeitungsvorkehrungen, d. h. z. B. über geeignete Auswertungs- und/oder Verarbeitungsalgorithmen, welche eine datenmäßige Auswertung bzw. Verarbeitung jeweiliger erfassungselementseitig gelieferter Erfassungssignale ermöglichen. Eine entsprechende Erfassungseinrichtung 13 kann jedoch auch identisch mit der bereits erwähnten Erfassungseinrichtung 12 zur Erfassung einer chemischen und/oder physikalischen Größe eingerichtet sein, als anhand jeweiliger erfasster chemischer und/oder physikalischer Größen Rückschlüsse auf ein auf den Auflagebereich 3 aufgelegtes Körperteil ziehbar sind.

Anhand von Fig. 5 ist ferner ersichtlich, dass die Auflageeinrichtung 1 eine, insbesondere in den Auflagekörper 2 integrierte, Erfassungseinrichtung 14 umfassen kann, welche zur Erfassung wenigstens einer physiologischen Größe, insbesondere des Blutdrucks, der Sauerstoffsättigung oder des Pulses, eines ein Körperteil auf den Auflagebereich 3 auflegenden Nutzers eingerichtet ist. Eine entsprechende Erfassungseinrichtung 14 kann ein oder mehrere, z. B. als Sensoren, insbesondere Blutdruck- bzw. Sauerstoffsättigungs- bzw. Pulssensoren, ausgebildete oder solche umfassende, Erfassungselemente (nicht gezeigt) umfassen, welche an oder in dem Auflagekörper 2 und/oder an oder in dem Auflagebereich 3 angeordnet oder ausgebildet sind. Entsprechende physiologische Größen können z. B. über den Kontaktbereich zwischen dem Auflagebereich 3 und dem jeweiligen auf den Auflagebereich 3 aufgelegten Körperteil erfasst werden. Eine entsprechende Erfassungseinrichtung 14 verfügt typischerweise über geeignete hardware- und/oder softwaremäßig implementierte Auswertungs- und/oder Verarbeitungsvorkehrungen, d. h. z. B. über geeignete Auswertungs- und/oder Verarbeitungsalgorithmen, welche eine datenmäßige Auswertung bzw. Verarbeitung jeweiliger erfassungselementseitig gelieferter Erfassungssignale ermöglichen.

Anhand von Fig. 5 ist ferner ersichtlich, dass die Auflageeinrichtung 1 eine Erfassungseinrichtung 15 umfassen kann, welche zur Erfassung einer Bewegung des Auflagekörpers 2 in wenigstens einem Bewegungsfreiheitsgrad, insbesondere relativ zu einem Untergrund, und/oder zur Erfassung einer Bewegung eines auf dem Auflagebereich 3 aufgelegten Körperteils in wenigstens einem Bewegungsfreiheitsgrad, insbesondere relativ zu dem Auflagekörper 2, eingerichtet ist. Eine entsprechende Erfassungseinrichtung 15 kann ein oder mehrere, z. B. als Sensoren, insbesondere Bewegungssensoren, ausgebildete oder solche umfassende, Erfassungselemente (nicht gezeigt) umfassen, welche an oder in dem Auflagekörper 2 und/oder an oder in dem Auflagebereich 3 angeordnet oder ausgebildet sind. Eine entsprechende Erfassungseinrichtung 15 verfügt typischerweise über geeignete hardware- und/oder softwaremäßig implementierte Auswertungs- und/oder Verarbeitungsvorkehrungen, d. h. z. B. über geeignete Auswertungs- und/oder Verarbeitungsalgorithmen, welche eine datenmäßige Auswertung bzw. Verarbeitung jeweiliger erfassungselementseitig gelieferter Erfassungssignale ermöglichen.

Wie durch den strichliert dargestellten Kasten angedeutet, können die Erfassungseinrichtungen 12 - 15 hardware- und/oder softwaremäßig auch in einer, gegebenenfalls übergeordneten, gemeinsamen Erfassungseinrichtung zusammengefasst sein.

Anhand von Fig. 5 ist ferner ersichtlich, dass die Auflageeinrichtung 1 eine der oder den Erfassungseinrichtung(en) 12 - 15 zuordenbare oder zugeordnete, insbesondere in den Auflagekörper 2 und/oder in den Auflagebereich 3 integrierte, Übertragungseinrichtung 16 umfassen kann, welche zur Übertragung jeweiliger Erfassungsinformationen an wenigstens einen Kommunikationspartner eingerichtet ist. Eine entsprechende Übertragungseinrichtung 16 kann entsprechende Erfassungsinformationen kabelgebunden oder kabellos an wenigstens einen Kommunikationspartner übertragen. Eine kabellose Übertragung kann z. B. vermittels einer Drahtlosverbindung bzw. eines Drahtlosnetzwerks über bekannte Industriestandards, wie z. B. Bluetooth, für die Datenübertragung erfolgen. Entsprechende Erfassungsinformationen können dabei unverschlüsselt oder verschlüsselt übertragen werden. Bei einem Kommunikationspartner kann es sich z. B. um ein mobiles Endgerät, wie z. B. einen mobilen Computer (Laptop), ein Smartphone, ein Tablet, eine Smartbrille, um ein stationäres Endgerät, wie z. B. einen stationären Computer (Desktop, Server), um eine Netzwerkanwendung bzw. um einen Netzwerkspeicher (Cloud), etc. handeln.

Anhand von Fig. 5 ist ferner ersichtlich, dass die Auflageeinrichtung 1 eine, insbesondere in den Auflagekörper 2 und/oder in den Auflagebereich 3 integrierte, Datenspeichereinrichtung 17 umfassen kann, welche zur Speicherung wenigstens einer vermittels einer Erfassungseinrichtung 12 - 15 erfassten Erfassungsinformation eingerichtet ist. Einer entsprechenden Datenspeichereinrichtung 17 ist typischerweise eine Datenübertragungsschnittstelle (nicht gezeigt) zugeordnet, über welche Daten kabelgebunden oder kabellos von der Datenspeichereinrichtung 17 gelesen und/oder über welche Daten in die Datenspeichereinrichtung 17 geschrieben werden können.

Anhand von Fig. 5 ist ferner ersichtlich, dass die Auflageeinrichtung 1 eine, insbesondere in den Auflagekörper 2 integrierte, Signalausgabeeinrichtung 18 umfassen kann, welche, insbesondere auf Grundlage wenigstens einer seitens einer Erfassungseinrichtung 12 - 15 erzeugten Erfassungsinformation, zur Erzeugung wenigstens eines, insbesondere akustischen und/oder optischen und/oder haptischen, Ausgabesignals eingerichtet ist. Über eine entsprechende Signalausgabeeinrichtung 18 können vermittels der Auflageeinrichtung 1 sonach bestimmte Ausgabesignale ausgegeben werden. Beispielsweise kann ein ein ordnungsgemäßes Auflegen eines Körperteils auf den Auflagebereich 3 anzeigendes Ausgabesignal ausgegeben werden. Ein entsprechendes Ausgabesignal kann beispielsweise auf Grundlage von von wenigstens einer Erfassungseinrichtung 12 - 15 gelieferten Erfassungsinformation, d. h. z. B. einer eine erfasste Kontaktierung des Auflagebereichs 3 durch ein Körperteil beschreibenden Erfassungsinformation, erzeugt werden bzw. worden sein. Eine entsprechende Signalausgabeeinrichtung 18 umfasst typischerweise wenigstens ein Signalausgabeelement (nicht gezeigt), welches je nach konkret auszugebendem Signal z. B. akustisches Signalausgabeelement, d. h. z. B. als Lautsprecher, als optisches Signalausgabeelement, d. h. z. B. als Anzeigeelement, Leuchtdiodenelement, etc., oder als haptisches Signalausgabeelement, d. h. z. B. als Vibrationselement, ausgebildet bzw. wenigstens ein solches umfassen kann.

Ein beispielhaft vermittels einer entsprechenden Signalausgabeeinrichtung 18 ausgebbares Ausgabesignal kann wenigstens eine Information betreffend eine nutzerseitige Handlung und/oder eine Bewertung einer nutzerseitigen Handlung, insbesondere an einen Nutzer, der Auflageeinrichtung 1 beinhalten. Derart ist es möglich, vermittels entsprechender entsprechende Informationen beinhaltender Ausgabesignale einen Nutzer bei der ordnungsgemäßen Nutzung der Auflageeinrichtung 1 zu unterstützen. Beispielsweise kann einem Nutzer durch entsprechende Ausgabesignale ausgegeben werden, dass die Auflageeinrichtung 1 zur Auflage eines Körperteils, gegebenenfalls auch welches Körperteils, bereit ist, dass eine ordnungsgemäße Auflage eines Körperteils erfolgt oder (noch) nicht erfolgt ist, wann bzw. dass eine bestimmte, z. B. für eine therapeutische Wirkung zweckmäßige Auflagedauer erreicht oder (noch) nicht erreicht ist, etc. Denkbar sind gleichermaßen einen Nutzer anspornende oder motivierende Ausgabesignale, d. h. Ausgabesignale, welche einen Nutzer, z. B. akustisch, optisch oder haptisch, anspornen bzw. motivieren, die Auflageeinrichtung 1 zu benutzen, eine bestimmte Auflageposition einzunehmen und/oder aufrechtzuerhalten, etc. In analoger Weise sind einen Nutzer bewertende, d. h. insbesondere lobende oder kritisierende, Ausgabesignale denkbar, welche einen Nutzer bzw. eine bestimmte Nutzerhandlung, z. B. akustisch, optisch oder haptisch, vor und/oder während und/oder nach Benutzung der Auflageeinrichtung 1 bewerten.

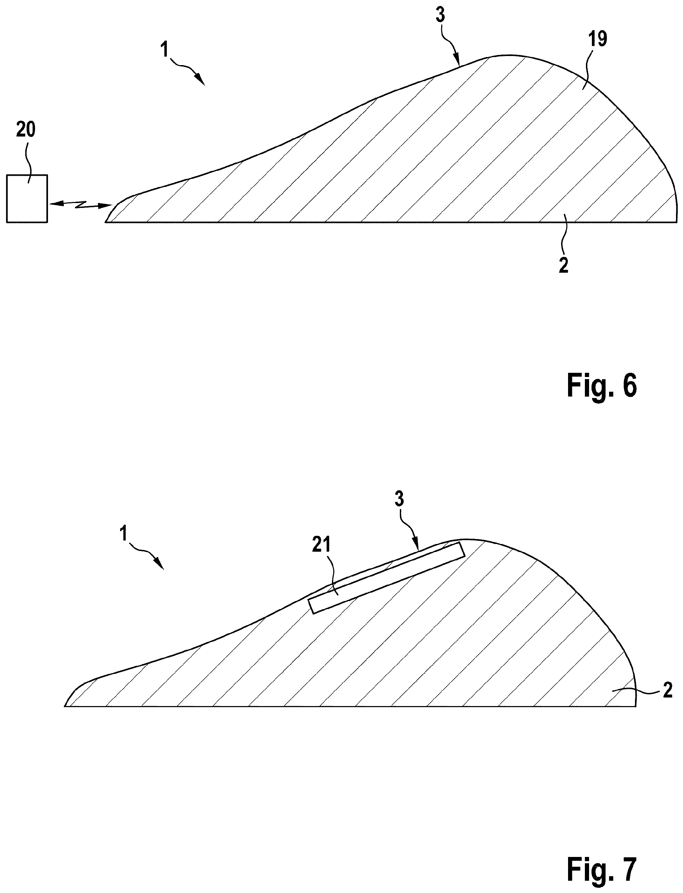

Fig. 6 zeigt eine Prinzipdarstellung einer Auflageeinrichtung 1 zur Auflage eines Körperteils (nicht gezeigt) eines Nutzers gemäß einem Ausführungsbeispiel in einer Seitenansicht.

Anhand von Fig. 6 ist ersichtlich, dass der Auflagekörper 2 in ein Eingabegerät 19 einer mit einem Eingabegerät steuerbaren Einrichtung 20, d. h. z. B. eines Endgeräts, wie z. B. eines Computers, eines Multimediagerät, etc. integriert oder als ein solches Eingabegerät 19 ausgebildet sein kann. Derart kann eine erhöhte Funktionsintegration erzielt werden, welche einem Nutzer zudem die Möglichkeit bietet, auch während der Auflage des Körperteils mit dem Körperteil bestimmte Handlungen, d. h. insbesondere Eingaben, Steuerungen, etc., vorzunehmen. Konkretes Beispiel für ein entsprechendes Eingabegerät 19 ist, wie in Fig. 6 gezeigt, ein, z. B. als (elektronische) Computermaus ausgebildetes, Eingabeelement zur Tätigung von durch den Pfeil angedeuteten Eingaben in einen Computer, ein, z. B. als Fernbedienung ausgebildetes, Eingabeelement zur Tätigung von Eingaben in ein Multimediagerät, wie z. B. einen Fernseher, eine Audioanlage, etc.

Fig. 7 zeigt eine Prinzipdarstellung einer Auflageeinrichtung 1 zur Auflage eines Körperteils (nicht gezeigt) eines Nutzers gemäß einem Ausführungsbeispiel in einer Seitenansicht.

Anhand von Fig. 7 ist ersichtlich, dass die Auflageeinrichtung 1 eine, insbesondere in den Auflagekörper 2 integrierte, Temperiereinrichtung 21 zur zumindest abschnittsweisen, gegebenenfalls vollständigen, Temperierung des Auflagebereichs 3 und/oder des Auflagekörpers 2 aufweisen bzw. umfassen kann. Die vermittels einer entsprechenden Temperiereinrichtung 21 mögliche statische oder dynamische Temperierung - hierunter ist grundsätzlich eine Beheizung als auch eine Kühlung zu verstehen - des Auflagebereichs 3 und/oder des Auflagekörpers 2 kann die gewünschte Entspannung eines jeweils aufgelegten Körperteils respektive einen durch die Auflage eines jeweiligen Körperteils erzielbaren therapeutischen Effekt unterstützen.

Eine entsprechende Temperiereinrichtung 21 kann eine wenigstens einen von einem Temperiermedium, d. h. z. B. einem Gas oder einer Flüssigkeit, durchströmbaren Temperierkanal umfassende Temperierkanalstruktur (nicht gezeigt) umfassen. Eine entsprechende Temperierkanalstruktur kann sich, insbesondere flächig, zumindest abschnittsweise, gegebenenfalls vollständig, durch den Auflagekörper 2 bzw. den Auflagebereich 3 erstrecken. Insbesondere kann sich eine entsprechende Temperierkanalstruktur, insbesondere konturnah, im Bereich der bzw. unterhalb der eine tatsächliche Auflagefläche des Auflagekörpers 2 bzw. des Auflagebereichs 3 bildenden (freiliegenden) Oberfläche des Auflagekörpers 2 bzw. des Auflagebereichs 3 erstrecken.

Alternativ oder ergänzend kann eine entsprechende Temperiereinrichtung 21 wenigstens ein elektrisch betreibbares Temperierelement (nicht gezeigt) umfassen. Ein entsprechendes elektrisch betreibbares Temperierelement kann z. B. als ein Heizdraht oder eine ein- oder mehrdimensionale Heizdrahtstruktur ausgebildet sein bzw. wenigstens eine(n) solche(n) umfassen. Ein entsprechendes Temperierelement kann sich, insbesondere flächig, zumindest abschnittsweise, gegebenenfalls vollständig, durch den Auflagekörper 2 bzw. den Auflagebereich 3 erstrecken. Insbesondere kann sich ein entsprechendes Temperierelement, insbesondere konturnah, im Bereich der bzw. unterhalb der eine tatsächliche Auflagefläche des Auflagekörpers 2 bzw. des Auflagebereichs 3 bildenden (freiliegenden) Oberfläche des Auflagekörpers 2 bzw. des Auflagebereichs 3 erstrecken.

Alternativ oder ergänzend kann eine entsprechende Temperiereinrichtung 21 wenigstens ein Temperierelement umfassen, welches nach Aktivierung eine, gegebenenfalls reversible, exotherme chemische Reaktion ausführt. Ein entsprechendes Temperierelement kann im Bereich der bzw. unterhalb der eine tatsächliche Auflagefläche des Auflagekörpers 2 bzw. des Auflagebereichs 3 bildenden (freiliegenden) Oberfläche des Auflagekörpers 2 bzw. des Auflagebereichs 3 angeordnet oder ausgebildet sein. Ein entsprechendes Temperierelement kann ein Aktivierungselement (nicht gezeigt), d. h. z. B. eine Lasche, einen Knopf, etc., umfassen, über welches eine Aktivierung des Temperierelements, d. h. eine Initiierung der exothermen Reaktion, möglich ist.

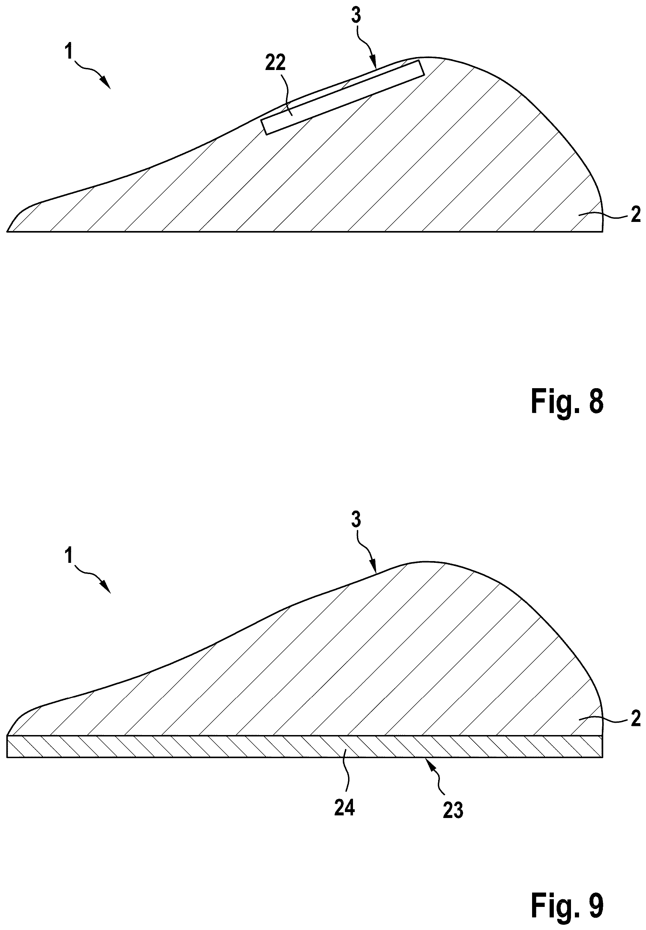

Fig. 8 zeigt eine Prinzipdarstellung einer Auflageeinrichtung 1 zur Auflage eines Körperteils (nicht gezeigt) eines Nutzers gemäß einem Ausführungsbeispiel in einer Seitenansicht.

Anhand von Fig. 8 ist ersichtlich, dass die Auflageeinrichtung 1 eine, insbesondere in den Auflagekörper 2 und/oder in den Auflagebereich 3 integrierte, Belüftungseinrichtung 22 zur zumindest abschnittsweisen, gegebenenfalls vollständigen, Belüftung eines auf der Auflageeinrichtung 3 aufzulegenden oder aufgelegten Körperteils aufweisen bzw. umfassen kann. Die vermittels einer entsprechenden Belüftungseinrichtung 22 mögliche statische oder dynamische Belüftung des Auflagekörpers 2 und/oder des Auflagebereichs 3 kann die gewünschte Entspannung eines aufgelegten Körperteils respektive einen durch die Auflage eines Körperteils erzielbaren therapeutischen Effekt unterstützen. Ebenso können die Auflageeigenschaften, insbesondere bei Auflegen eines Körperteils über einen längeren Zeitraum, verbessert werden.

Eine entsprechende Belüftungseinrichtung 22 kann eine wenigstens einen von einem Belüftungsmedium, d. h. z. B. einem Gas, durchströmbaren Belüftungskanal umfassende Belüftungskanalstruktur (nicht gezeigt) umfassen. Eine entsprechende Belüftungskanalstruktur kann sich, insbesondere flächig, zumindest abschnittsweise, gegebenenfalls vollständig, durch den Auflagekörper 2 bzw. den Auflagebereich 3 erstrecken. Insbesondere kann sich eine entsprechende Belüftungskanalstruktur, insbesondere konturnah, im Bereich der bzw. unterhalb der eine tatsächliche Auflagefläche 3 des Auflagekörpers 2 bzw. des Auflagebereichs 3 bildenden (freiliegenden) Oberfläche des Auflagekörpers 2 bzw. des Auflagebereichs 3 erstrecken.

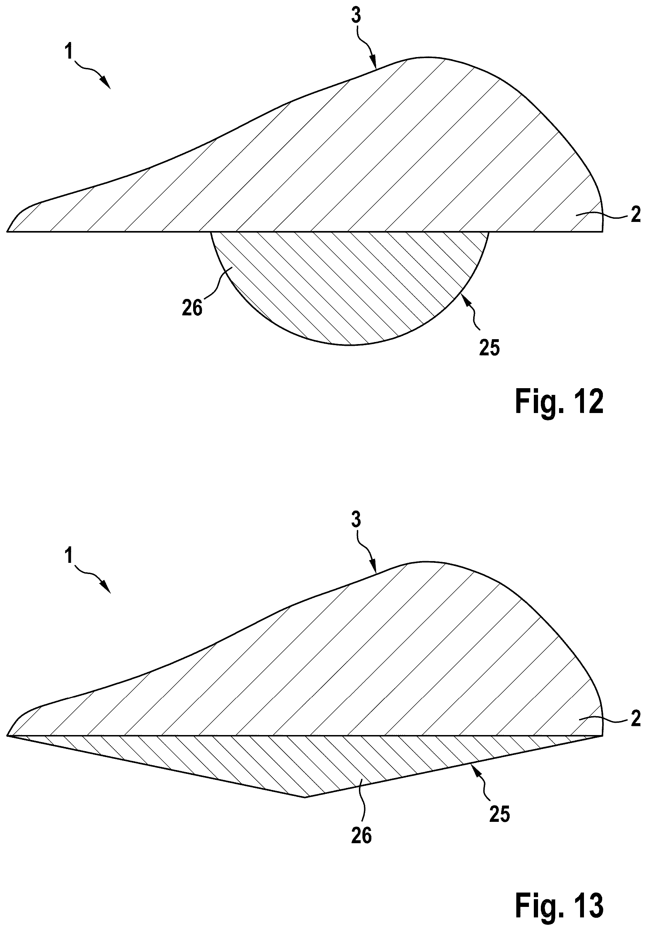

Die Fig. 9 - 13 zeigen jeweils eine Prinzipdarstellung einer Auflageeinrichtung 1 zur Auflage eines Körperteils (nicht gezeigt) eines Nutzers gemäß weiteren Ausführungsbeispielen in einer Seitenansicht.

Anhand von Fig. 9 ist ersichtlich, dass die Auflageeinrichtung 1 eine Befestigungseinrichtung 23 zur zeitweisen Befestigung der Auflageeinrichtung 1 an oder auf einem Drittgegenstand, insbesondere an oder auf einem an einem Körper eines Nutzers der Auflageeinrichtung 1 zu tragenden Drittgegenstand, aufweisen bzw. umfassen. Die Auflageeinrichtung 1 kann derart auch an Drittgegenständen, d. h. insbesondere mobilen bzw. portablen Drittgegenständen, befestigt werden. Die Befestigung der Auflageeinrichtung 1 an entsprechenden Drittgegenständen kann insbesondere derart sein, dass die Auflageeinrichtung 1 in einer Ausrichtung und/oder Position, in welcher eine gewünschte Ausrichtung eines auf dem Auflagebereich 3 aufgelegten Körperteils möglich ist und aufrechterhalten werden kann. Ein konkretes Beispiel für einen Drittgegenstand ist ein an einem Körper eines Nutzers zu tragender Drittgegenstand, wie z. B. ein Kleidungsstück. Die Auflageeinrichtung 1 kann sonach an oder in einem Drittgegenstand, wie z. B. einem Kleidungsstück, befestigbar sein. Beispielsweise ist es denkbar, dass die Auflageeinrichtung 1 in einer Tasche eines Kleidungsstücks, wie z. B. eines am Oberkörper eines Trägers bzw. einer Trägerin zu tragenden Kleidungsstücks, wie z. B. einer Jacke, eines Pullovers, etc., oder eines am Unterkörper eines Trägers bzw. einer Trägerin zu tragenden Kleidungsstücks, wie z. B. einer Hose, eines Rocks, etc., befestigbar ist. Die Befestigungseinrichtung 23 kann ein oder mehrere rein schematisch angedeutete Befestigungselemente 24 umfassen, welche zur (beschädigungs- bzw. zerstörungsfreien) Befestigung der Auflageeinrichtung 1 an einem entsprechenden Drittgegenstand eingerichtet sind. Entsprechende Befestigungselemente 24 können z. B. eine form- und/oder kraftschlüssige Befestigung der Auflageeinrichtung 1 an einem entsprechenden Drittgegenstand ermöglichen. Konkretes Beispiel für entsprechende Befestigungselemente 24 sind demnach Haken- und/oder Verschlaufungselemente (Klettverschlusselemente).

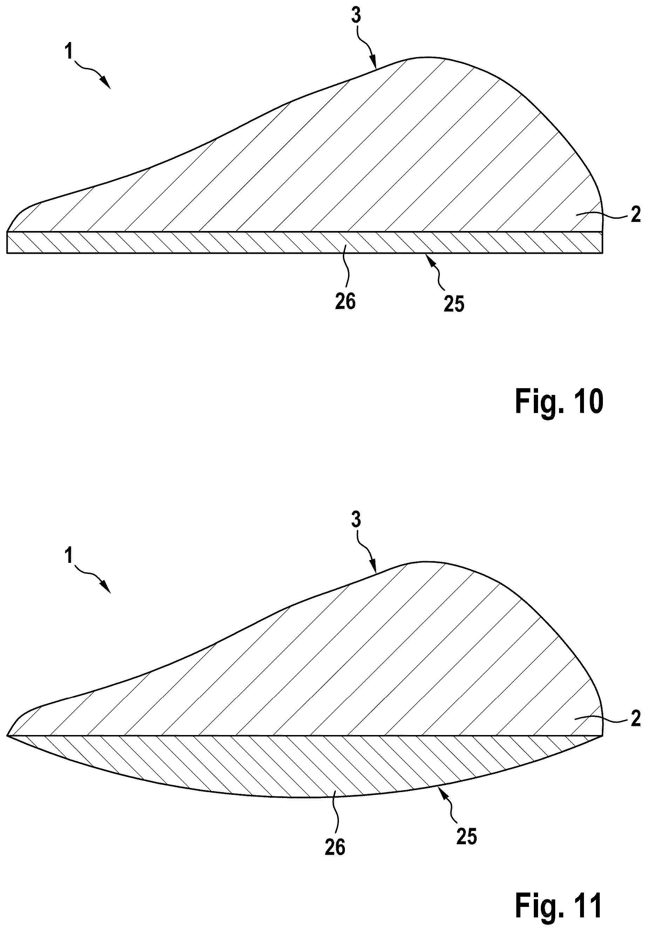

Anhand von Fig. 10 ist ersichtlich, dass die Auflageeinrichtung 1 einen Auflagekörper 2 mit einen Lagerungsabschnitt 25 zur Lagerung des Auflagekörpers 2 auf einem Untergrund bzw. einer Unterlage aufweisen kann. Über einen entsprechenden Lagerungsabschnitt 25 kann - je nach Ausführung - eine stabile Lagerung des Auflagekörpers 2 auf einem Untergrund bzw. einer Unterlage, wie z. B. einer Tischfläche, realisiert werden.

Ein entsprechender Lagerungsabschnitt 25 kann mit wenigstens einem eine rutschfeste Auflage des Auflagekörpers 2 auf einem Untergrund bzw. einer Unterlage schaffenden Material bzw. einer entsprechenden Materialstruktur versehen sein bzw. ein(e) solche(s) umfassen. Ein entsprechendes Material bzw. eine entsprechende Materialstruktur kann z. B. deshalb zweckmäßig sein, um eine stabile Auflage des Auflagekörpers 2 auf einem Untergrund bzw. einer Unterlage zu ermöglichen. Ein entsprechendes Material bzw. eine entsprechende Materialstruktur kann z. B. aus einem elastomeren Material bzw. einer elastomeren Materialstruktur gebildet sein bzw. wenigstens eine elastomeres Material umfassen. In Frage kommen grundsätzlich sowohl natürliche als auch synthetische elastomere Materialien bzw. Materialstrukturen. **In** fertigungstechnischer Hinsicht vorteilhaft können z. B. thermoplastische Elastomere verwendet werden.

Ein entsprechender Lagerungsabschnitt 25 kann mit wenigstens einem reversibel verformbaren Material, insbesondere einem reversibel verformbaren weich-elastischen Material, bzw. einer reversibel verformbaren Materialstruktur, insbesondere einer reversibel verformbaren weich-elastischen Materialstruktur versehen sein bzw. ein(e) solche(s) umfassen. Ein entsprechendes Material bzw. eine entsprechende Materialstruktur kann z. B. deshalb zweckmäßig sein, als etwaig erforderliche Änderungen der Ausrichtung des Auflagekörpers 2 auf einem Untergrund bzw. einer Unterlage, insbesondere relativ zu einer Neutral- oder Referenzausrichtung, ermöglichen, welche z. B. Nutzern mit spastischen Körperteilen bzw. Gliedmaßen das Benutzen der Auflageeinrichtung 1 erleichtern bzw. ermöglichen kann; dies gilt insbesondere dann, wenn die jeweilige Spastik oder eine sonstige anatomische Fehlstellung eine besondere Ausrichtung des Auflagekörpers 2, insbesondere relativ zu einer Neutral- oder Referenzausrichtung, erfordert, um das Körperteil, insbesondere schmerzfrei, auf den Auflagebereich 3 aufzulegen. Ein entsprechendes Material bzw. eine entsprechende Materialstruktur kann z. B. aus einem elastomeren oder zellularen Material bzw. einer elastomeren bzw. zellularen Materialstruktur gebildet sein bzw. wenigstens eine elastomeres Material umfassen. In Frage kommen grundsätzlich sowohl natürliche als auch synthetische elastomere bzw. zellulare Materialien bzw. Materialstrukturen. **In** fertigungstechnischer Hinsicht vorteilhaft können z. B., gegebenenfalls geschäumte, thermoplastische Elastomere verwendet werden.

Ein entsprechender Lagerungsabschnitt 25 kann, wie in den Fig. 10 - 13 beispielhaft gezeigt, ein, insbesondere nicht plan auf einem planen Untergrund bzw. einer planen Unterlage aufliegendes, Lagerungsabschnittelement 26 aufweisen, welches eine in wenigstens einem Bewegungsfreiheitsgrad bewegbare, insbesondere schwenkbewegbare, Lagerung des auf dem Untergrund gelagerten Auflagekörpers 2 relativ zu dem Untergrund bzw. der Unterlage ermöglicht. Derart kann der Auflagekörper 2 relativ zu einem Untergrund bzw. einer Unterlage bewegt werden, was vorteilhaft sein kann, um eine möglichst entspannte Positionierung eines jeweiligen Körperteils sowie übergeordneter Körperteile zu realisieren. Beispielsweise kann eine im Vergleich zu einer Referenzausrichtung veränderliche Ausrichtung des Auflagekörpers 2 eine (noch) entspannendere bzw. entspanntere Auflage eines Körperteils ermöglichen. Ein entsprechendes Lagerungsabschnittelement 26 kann, wie die Fig. 10 - 13 zeigen, z. B. eine ein- oder mehreckige, runde, rundliche oder spitze Geometrie aufweisen. Ebenso kann eine bewegbare Lagerung des Auflagekörpers 2 Trainingsmöglichkeiten, z. B. zum Muskelaufbau, eröffnen, als durch das Bewegen des Auflagekörpers 2 relativ zu dem Untergrund bzw. der Unterlage bestimmte, mit einem Trainingseffekt einhergehende Bewegungsabläufe vollzogen werden können.

Ein entsprechendes Lagerungsabschnittelement 26 kann auch als gesondertes Bauelement ausgebildet sein und mit dem Auflagekörper 2 bedarfsweise unter Ausbildung der Auflageeinrichtung 1, gegebenenfalls (beschädigungs- bzw. zerstörungsfrei) lösbar, verbindbar oder verbunden sein. Ein entsprechendes, z. B. kugelförmiges, halbkugelförmiges, zylinderförmiges oder halbzylinderförmiges oder pyramidenförmiges, Lagerungsabschnittelement 26 kann z. B. über ein Klett- bzw. Wechselschlusssystem mit dem Auflagekörper 2 verbunden sein.

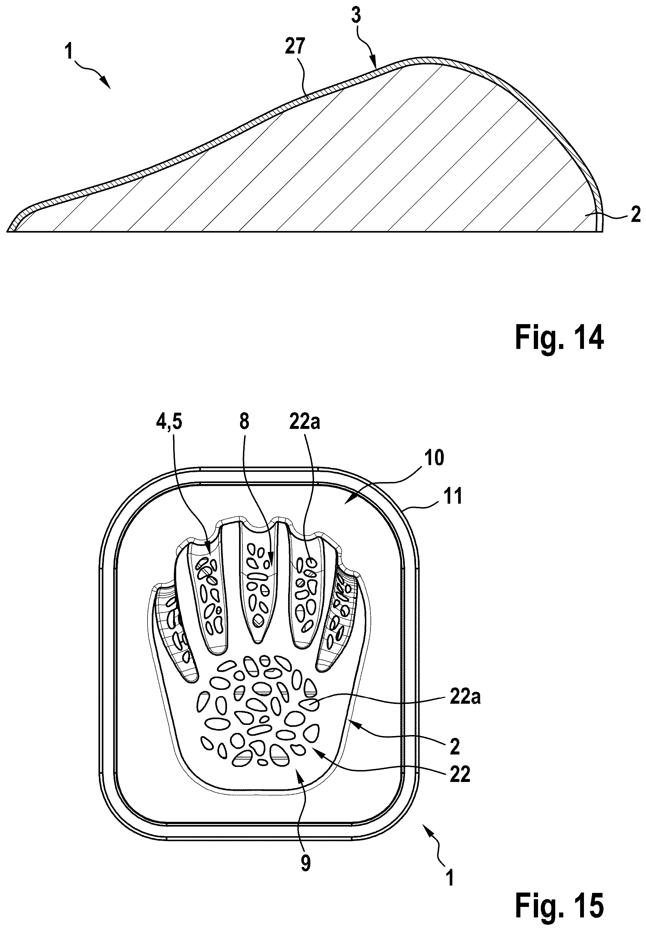

Fig. 14 zeigt eine Prinzipdarstellung einer Auflageeinrichtung 1 zur Auflage eines Körperteils (nicht gezeigt) eines Nutzers gemäß einem weiteren Ausführungsbeispiel in einer Seitenansicht.

Anhand von Fig. 14 ist ersichtlich, dass die Auflageeinrichtung 1 ein, insbesondere (beschädigungs- bzw. zerstörungsfrei) lösbar, auf dem Auflagebereich 3, diesen zumindest abschnittsweise, gegebenenfalls vollständig, abdeckend auflegbares bzw. aufgelegtes Auflageelement 27 umfassen kann. Ein entsprechendes Auflageelement 27 kann insbesondere unter hygienischen Gesichtspunkten zweckmäßig sein, als der Auflagebereich 3 respektive der Auflagekörper 2 vor einem direkten Kontakt mit dem jeweiligen Körperteil und somit z. B. vor Ablagerungen der Haut, Schweiß, etc. geschützt ist. Ein entsprechendes Auflageelement 27 kann aus einem elastisch-flexiblen Material bzw. einer elastisch-flexiblen Materialstruktur gebildet sein, sodass das Auflageelement 27 die Passform des Auflagebereichs 3 nicht beeinträchtigt. Ein entsprechendes Auflageelement 27 kann aus einem, gegebenenfalls (ab)waschbaren, natürlichen und/oder synthetischen textilen Material oder einer natürlichen und/oder synthetischen textilen Materialstruktur ausgebildet sein. Lediglich beispielhaft ist auf Leder- oder Kunststoffmaterialien zu verweisen.

Fig. 15 zeigt eine Prinzipdarstellung einer Auflageeinrichtung 1 zur Auflage eines Körperteils (nicht gezeigt) eines Nutzers gemäß einem weiteren Ausführungsbeispiel in einer Aufsicht.

Das in Fig. 15 gezeigte Ausführungsbeispiel ist ebenso mit einer Belüftungseinrichtung 22 zur Belüftung des Auflagebereichs 3 und/oder des Auflagekörpers 2 und/oder eines auf der Auflageeinrichtung 1 aufzulegenden oder aufgelegten Körperteils versehen.

Anhand von Fig. 15 ist ersichtlich, dass eine entsprechende Belüftungskanalstruktur einer Belüftungseinrichtung 22 durch ein oder mehrere vordefiniert konfigurierte bionische Geometrien ausgebildet sein kann. Die Belüftungskanalstruktur ist in Fig. 15 durch entsprechende Öffnungen 22a jeweiliger Belüftungskanäle angedeutet. Entsprechende Öffnungen 22a können z. B. durch bohrungsartige bzw. -förmige Ausnehmungen gebildet sein. Ersichtlich können entsprechende Belüftungskanäle im Bereich des Grundabschnitt 6 als auch im Bereich wenigstens eines Auflageabschnitts 4 ausgebildet sein. Selbstverständlich ist es jedoch denkbar, dass entsprechende Belüftungskanäle nur im Bereich des Grundabschnitts 6 oder nur im Bereich wenigstens eines Auflageabschnitts 4 ausgebildet sind.

Einzelne, mehrere oder sämtliche Belüftungskanäle können grundsätzlich von deren Anordnung miteinander kommunizieren. Entsprechende bionische Geometrien können eine Belüftung eines auf der Auflageeinrichtung 1 aufzulegenden oder aufgelegten Körperteils unterstützen bzw. verbessern. Grundsätzlich ist es sonach möglich in dem Auflagekörper 2, vordefiniert konfigurierte bionische Geometrien zur Ausbildung einer entsprechenden Belüftungskanalstruktur auszubilden.

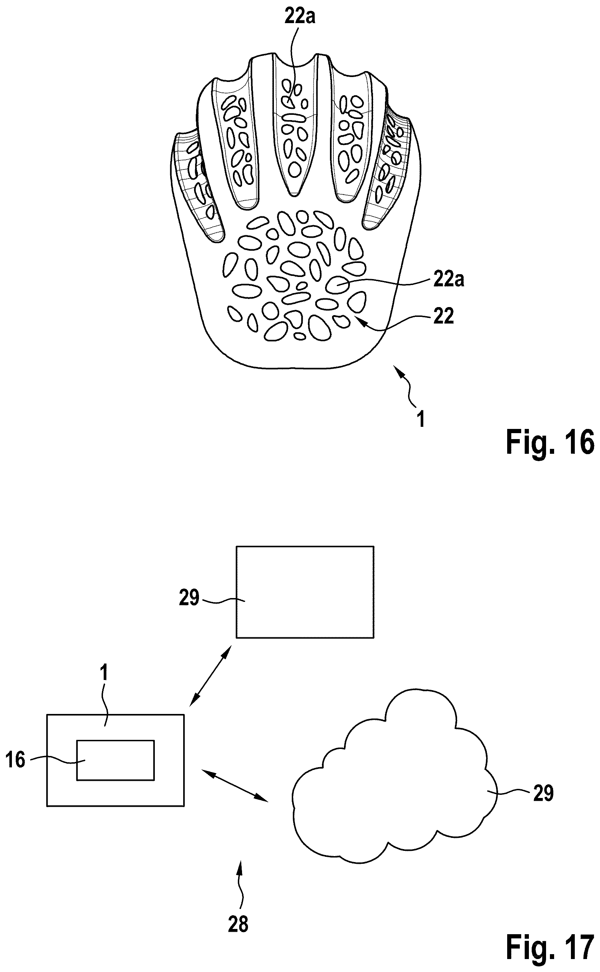

Fig. 16 zeigt eine Prinzipdarstellung einer Auflageeinrichtung 1 zur Auflage eines Körperteils (nicht gezeigt) eines Nutzers gemäß einem weiteren Ausführungsbeispiel in einer Aufsicht.

Das in Fig. 16 gezeigte Ausführungsbeispiel unterscheidet sich von dem in Fig. 15 gezeigten Ausführungsbeispiel dadurch, dass die Auflageeinrichtung 1 keine Grund- bzw. Trägerplatte 10 und somit keine Trägereinrichtung zum Tragen der Auflageeinrichtung 1 aufweist. Dies gilt, wie angedeutet, grundsätzlich für alle Ausführungsbeispiele der Auflageeinrichtung 1; mithin können alle Ausführungsbeispiele der Auflageeinrichtung 1 ohne entsprechende Trägereinrichtung zum Tragen der Auflageeinrichtung 1 ausgeführt sein.

Wenngleich in den Fig. nicht gezeigt, kann die Auflageeinrichtung 1 eine dem Auflagekörper 2 und/oder dem Auflagebereich 3 zuordenbare oder zugeordnete Fixiereinrichtung zur zeitweisen Fixierung eines auf den Auflagebereich 3 aufgelegten Körperteils, insbesondere in einer bestimmungsgemäßen Auflageposition des Körperteils auf dem Auflagebereich 3, umfassen. Eine entsprechende Fixierung kann eine gewünschte Ausrichtung eines auf dem Auflagebereich 3 aufgelegten Körperteils ermöglichen und aufrechterhalten. Derart kann ein vermittels der Auflageeinrichtung 1 realisierbarer Entspannungseffekt bzw. therapeutischer Effekt unterstützt werden. Eine entsprechende Fixiereinrichtung kann eine Fixierung eines jeweiligen Körperteils unmittelbar an dem Auflagekörper 2 respektive dem Auflagebereich 3 ermöglichen; dies kann z. B. über ein an dem Auflagekörper 2 oder dem Auflagebereich 3 anbringbares oder angebrachtes Fixierelement, wie z. B. einen Gurt, eine Schlaufe, etc., realisiert sein, welches das jeweilige Körperteil nach Anlegen zumindest abschnittsweise, gegebenenfalls vollständig, umschließt, sodass das Körperteil an dem Auflagekörper 2 respektive dem Auflagebereich 3 fixiert ist. Denkbar ist auch eine schlauchartige bzw. -förmige Fixiereinrichtung, d. h. z. B. ein z. B. aus einem Textil gebildeter Fixierschlauch, welcher über die Auflageeinrichtung und das auf dieser aufzulegende bzw. aufgelegte Körperteil zu ziehen ist und derart eine gewünschte Ausrichtung eines auf dem Auflagebereich aufgelegten Körperteils ermöglicht und aufrechterhält. Ebenso denkbar ist eine Fixiereinrichtung, welche eine Fixierung eines übergeordneten Körperteils, d. h. z. B. eines Arms, wenn das zu fixierende Körperteil eine Hand ist, in einer bestimmten Ausrichtung ermöglicht.

Für alle Ausführungsbeispiele gilt, dass der Auflagebereich 3 und/oder der Auflagekörper 2 aus wenigstens einem Schaummaterial, d. h. insbesondere aus einem expandierten Partikelschaummaterial, wie z. B. einem expandierten Kunststoffmaterial, gebildet sein können. Entsprechende Schaummaterialien reduzieren das Gewicht des Auflagekörpers 2 bzw. des Auflagebereichs 3. Entsprechende Schaummaterialien können zudem angenehme haptische Eigenschaften des Auflagekörpers 2 bzw. des Auflagebereichs 3 bedingen. Weitere Vorteile entsprechender Schaummaterialien sind deren gut beherrschbare kosteneffiziente Verarbeitung.

Für alle Ausführungsbeispiele gilt ferner, dass der Auflagebereich 3 und/oder der Auflagekörper 2 aus einem magnetisierbaren oder magnetischen Material gebildet oder wenigstens ein solches umfassen können. Magnetisierbare oder magnetische Materialien, wie z. B. entsprechende ferromagnetische Metalle, können sich positiv auf den Blutfluss durch ein entsprechendes Körperteil auswirken, was therapeutische Effekte begünstigen kann. Magnetisierbare oder magnetische Materialien können als Vollmaterial oder in Form von Partikeln vorliegen.

Für alle Ausführungsbeispiele gilt ferner, dass der Auflagebereich 3 und/oder der Auflagekörper 2 alternativ oder ergänzend aus einem antibakteriellen Material gebildet oder wenigstens ein solches umfassen können. Antibakterielle Materialien, wie z. B. antibakterielle Metalle, können sich positiv auf die hygienischen Eigenschaften des Auflagebereichs 3 und/oder des Auflagekörpers 2 auswirken. Antibakterielle Materialien können als Vollmaterial oder in Form von Partikeln vorliegen.

Im Hinblick auf die Herstellung der Auflageeinrichtung 1, d. h. des Auflagekörpers 2 und/oder des Auflagebereichs 3, gilt, dass grundsätzlich sämtliche, insbesondere urformenden und/oder formgebenden, Fertigungsverfahren in Betracht kommen.

Insbesondere kommen sowohl additive (aufbauende) Fertigungsverfahren als auch subtraktive (abtragende) Fertigungsverfahren, d. h. insbesondere spanabhebende Fertigungsverfahren, in Betracht.

Fig. 17 zeigt eine Prinzipdarstellung eines Kommunikationssystems 28 gemäß einem Ausführungsbeispiel. Das Kommunikationssystem 28 umfasst wenigstens eine Auflageeinrichtung 1, welche eine Übertragungseinrichtung 16, welche zur Übertragung einer Information an wenigstens einen Kommunikationspartner 29 und/oder zum Empfang einer von wenigstens einem Kommunikationspartner 29 übertragenen Information eingerichtet ist, sowie wenigstens einen Kommunikationspartner 29, welcher zum Empfang einer von der wenigstens einen Auflageeinrichtung 1 übertragenen Information und/oder zur Übertragung einer Information an die wenigstens eine Auflageeinrichtung 1 eingerichtet ist.

Bei dem Kommunikationspartner 29 kann es sich z. B. um ein mobiles Endgerät, wie z. B. einen mobilen Computer (Laptop), ein Smartphone, ein Tablet, eine Smartbrille, um ein stationäres Endgerät, wie z. B. einen stationären Computer (Desktop, Server), um eine Netzwerkanwendung bzw. um einen Netzwerkspeicher (Cloud), etc. handeln.

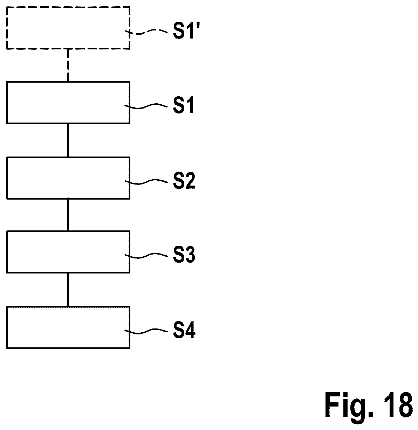

Fig. 18 zeigt eine Prinzipdarstellung eines Verfahrens zur Herstellung einer Auflageeinrichtung 1 zur Aufnahme eines Körperteils eines Nutzers gemäß einem Ausführungsbeispiel. Das Verfahren umfasst die folgenden Schritte:
Schritt S1, in welchem ein Bereitstellen einer die Abmessungen eines auf einer herzustellenden Auflageeinrichtung aufzulegenden Körperteils zumindest abschnittsweise, gegebenenfalls vollständig, beschreibenden Körperteilabmessungsinformation erfolgt.
Schritt S2, in welchem ein Bereitstellen, z. B. in einer Datenspeichereinrichtung, mehrerer Auflageeinrichtungsabmessungsinformationen, wobei jede Auflageeinrichtungsabmessungsinformation wenigstens eine wenigstens einer Abmessung eines Körperteils zugeordnete vordefinierte Abmessung einer auf Grundlage der jeweiligen Auflageeinrichtungsabmessungsinformation herstellbaren bzw. herzustellenden Auflageeinrichtung beschreibt, erfolgt.
Schritt S3, in welchem ein Auswählen einer Auflageeinrichtungsabmessungsinformation auf Grundlage der bereitgestellten Körperteilabmessungsinformation, erfolgt.
Schritt S4, in welchem ein Herstellen einer Auflageeinrichtung 1 auf Grundlage von die ausgewählte Auflageeinrichtungsabmessungsinformation beinhaltenden oder beschreibenden Daten erfolgt.

Entsprechende Daten, auf Grundlage welche die Auflageeinrichtung 1 verfahrensgemäß hergestellt wird, können sonach Herstellungsdaten einer zur Herstellung der jeweiligen Auflageeinrichtung 1 verwendbaren bzw. verwendeten Herstellungsanlage beinhalten oder in solche Herstellungsdaten umgewandelt werden.

Das Auswählen kann verfahrensgemäß durch Abgleich oder Vergleich der bereitgestellten Körperteilabmessungsinformation mit jeweiligen jeweiligen Auflageeinrichtungsabmessungsinformationen zugeordneten Körperteilabmessungsinformationen erfolgen, wobei eine Auflageeinrichtungsabmessungsinformation ausgewählt werden kann, deren zugeordnete Körperteilabmessungsinformation der bereitgestellten Körperteilabmessungsinformation gleicht oder, insbesondere im Rahmen eines vordefinierbaren oder vordefinierten Toleranzbereichs, ähnelt.

Eine verfahrensgemäß bereitzustellende bzw. bereitgestellte Körperteilabmessungsinformation kann durch, insbesondere optisches, Erfassen eines auf der Auflageeinrichtung 1 aufzulegenden Körperteils eines Nutzers erfolgen. Verfahrensgemäß kann sonach ein, insbesondere optisches, Erfassen eines auf der Auflageeinrichtung aufzulegenden Körperteils eines Nutzers und ein Erzeugen einer bereitzustellenden Körperteilabmessungsinformation auf Grundlage einer durch das Erfassen erzeugten Erfassungsinformation erfolgen (Schritt S1'). Das Erfassen kann, wie angedeutet, insbesondere optisch erfolgen, sodass ein Erfassen z. B. vermittels einer Erfassungseinrichtung in Form einer Kamera- oder Videoeinrichtung durchgeführt werden kann. Eine entsprechende Kamera- oder Videoeinrichtung kann Bestandteil eines nutzer- oder herstellerseitigen, insbesondere mobilen, Endgeräts, wie z. B eines mobilen Computers (Laptop), eines Smartphones, eines Tablets, einer Smartbrille, etc., bilden.

Einzelne, mehrere oder sämtliche Merkmale eines Ausführungsbeispiels können mit einzelnen, mehreren oder sämtlichen Merkmalen wenigstens eines weiteren Ausführungsbeispiels kombiniert werden.

Ein wesentlicher Aspekt der Erfindung betrifft eine Auflageeinrichtung zur zumindest abschnittsweisen, gegebenenfalls vollständigen, Auflage wenigstens eines Körperteils eines Nutzers, umfassend einen Auflagekörper, welcher wenigstens einen Auflagebereich zur zumindest abschnittsweisen, gegebenenfalls vollständigen, Auflage wenigstens eines Körperteils eines Nutzers aufweist, wobei zumindest der Auflagebereich zumindest abschnittsweise, gegebenenfalls vollständig, eine auf Grundlage einer Körperteilabmessungsinformation, welche die Abmessungen eines auf die Auflageeinrichtung aufzulegenden Körperteils zumindest abschnittsweise, gegebenenfalls vollständig, beschreibt, aus einer Mehrzahl an vordefinierten Auflageeinrichtungsabmessungsinformationen, wobei jede Auflageeinrichtungsabmessungsinformation wenigstens einer Abmessung eines Körperteils zugeordnete vordefinierte Abmessungen einer auf Grundlage der jeweiligen Auflageeinrichtungsabmessungsinformation herstellbaren Auflageeinrichtung beschreibt, ausgewählten Auflageeinrichtungsabmessungsinformation beschreibenden Daten ausgebildeten oder erzeugten vordefiniert konfiguriert ausgebildete geometrisch-konstruktive Gestaltung oder Formgebung aufweist.

## Patentansprüche

1. Auflageeinrichtung (1) zur zumindest abschnittsweisen, gegebenenfalls vollständigen, Auflage wenigstens eines Körperteils, insbesondere einer Hand, eines Nutzers, umfassend einen Auflagekörper (2), welcher wenigstens einen Auflagebereich (3) zur zumindest abschnittsweisen, gegebenenfalls vollständigen, Auflage wenigstens eines Körperteils eines Nutzers aufweist, wobei der Auflagekörper (2) oder der Auflagebereich (3) insbesondere eine einem auf der Auflageeinrichtung (1) aufzulegenden Körperteil nachempfunden vordefiniert konfiguriert ausgebildete Grundform aufweist, **dadurch gekennzeichnet, dass** zumindest der Auflagebereich (3) zumindest abschnittsweise, gegebenenfalls vollständig, eine vordefiniert konfiguriert ausgebildete geometrisch-konstruktive Gestaltung oder Formgebung aufweist, welche
auf Grundlage von durch eine Auflageeinrichtungsabmessungsinformation beschriebenen Daten ausgebildet bzw. erzeugt ist, wobei
die die Grundlage der für die Ausbildung bzw. Erzeugung des Auflagebereichs verwendeten Daten bildende Auflageeinrichtungsabmessungsinformation aus einer Mehrzahl an vordefinierten Auflageeinrichtungsabmessungsinformationen ausgewählt ist, wobei
die Auswahl der Auflageeinrichtungsabmessungsinformation aus der Mehrzahl an Auflageeinrichtungsabmessungsinformationen auf Grundlage einer Körperteilabmessungsinformation, welche die Abmessungen eines auf die Auflageeinrichtung aufzulegenden Körperteils zumindest abschnittsweise, gegebenenfalls vollständig, beschreibt, erfolgt, ferner umfassend eine, insbesondere in den Auflagekörper (2) und/oder in den Auflagebereich (3) integrierte, Belüftungseinrichtung (22) zur zumindest abschnittsweisen, gegebenenfalls vollständigen, Belüftung des Auflagebereichs (3) und/oder des Auflagekörpers (2) und/oder eines auf der Auflageeinrichtung (1) aufzulegenden oder aufgelegten Körperteils.

2. Auflageeinrichtung nach Anspruch 1, **dadurch gekennzeichnet, dass** die Belüftungseinrichtung eine wenigstens einen von einem Belüftungsmedium, d. h. z. B. einem Gas, durchströmbaren Belüftungskanal umfassende Belüftungskanalstruktur umfasst.

3. Auflageeinrichtung nach Anspruch 1 oder 2, **dadurch gekennzeichnet, dass** die Belüftungskanalstruktur sich, insbesondere flächig, zumindest abschnittsweise, gegebenenfalls vollständig, durch den Auflagekörper bzw. den Auflagebereich erstreckt.

4. Auflageeinrichtung nach einem der vorhergehenden Ansprüche, **dadurch gekennzeichnet, dass** die Belüftungskanalstruktur durch ein oder mehrere vordefiniert konfigurierte bionische Geometrien ausgebildet ist.

5. Auflageeinrichtung nach einem der vorhergehenden Ansprüche, **dadurch gekennzeichnet, dass** die Belüftungskanalstruktur durch Öffnungen, welche insbesondere durch bohrungsartige bzw. -förmige Ausnehmungen gebildet sind, jeweiliger Belüftungskanäle gebildet ist, wobei die Belüftungskanäle im Bereich eines Grundabschnitt als auch im Bereich wenigstens eines Auflageabschnitts ausgebildet sein.

6. Auflageeinrichtung nach einem der vorhergehenden Ansprüche, **dadurch gekennzeichnet, dass** der Auflagekörper (2) oder der Auflagebereich (3) eine bestimmte Anzahl und/oder eine bestimmte Anordnung an vordefiniert konfiguriert ausgebildeten Auflageabschnitten (4) aufweisen, welche der Anzahl und/oder Anordnung der Gliedmaßenabschnitte, insbesondere der Finger oder Zehen, eines jeweiligen auf der Auflageeinrichtung (1) aufzulegenden Körperteils eines Nutzers entspricht, wobei optional der Auflagekörper (2) oder der Auflagebereich (3) wenigstens zwei, insbesondere wenigstens drei, insbesondere wenigstens vier, vordefiniert konfiguriert ausgebildete Fingerauflageabschnitte in paralleler Anordnung, insbesondere in im Wesentlichen paralleler Anordnung, aufweist, welche der Anzahl und Anordnung der Finger einer jeweiligen auf der Auflageeinrichtung (1) aufzulegenden Hand eines Nutzers entspricht, wobei
optional die Fingerauflageabschnitte sich in einer ersten Raumrichtung, insbesondere einer distalen Raumrichtung bezogen auf eine auf der Auflageeinrichtung (1) aufgelegte Hand, ersteckend angeordnet und/oder ausgerichtet sind, wobei.
optional wenigstens ein sich quer zu den Fingerauflageabschnitten erstreckend angeordneter oder ausgerichteter weiterer Auflageabschnitt (31) zur Aufnahme eines Endbereichs eines Mittelhandbereichs einer auf der Auflageeinrichtung (1) aufzulegenden Hand eines Nutzers vorhanden ist, wobei
optional die Fingerauflageabschnitte, insbesondere unmittelbar, in den weiteren Auflageabschnitt übergehen.

7. Auflageeinrichtung nach einem der vorhergehenden Ansprüche, **dadurch gekennzeichnet, dass** der Auflagekörper (2) oder der Auflagebereich (3) einen Grundabschnitt umfasst, welcher eine oder mehrere, insbesondere relativ zu einer ungewölbten Grundebene, insbesondere einer Hand, gegebenenfalls einer Mittelhand nachempfunden, gewölbt ausgebildete Wölbungsabschnitte (8, 9) aufweist, wobei optional die Fingerauflageabschnitte und/oder der wenigstens eine weitere Auflageabschnitt (3) in wenigstens einem, insbesondere ringsegmentartig oder -förmig, gewölbt ausgebildeten Wölbungsabschnitt (8, 9) ausgebildet sind.

8. Auflageeinrichtung nach einem der vorhergehenden Ansprüche, **gekennzeichnet durch** eine, insbesondere in den Auflagebereich (3) und/oder den Auflagekörper (2) integrierte, Erfassungseinrichtung (23), welche zur Erfassung wenigstens einer chemischen und/oder physikalischen Größe des Auflagebereichs (3) und/oder des Auflagekörpers (2) und/oder eines auf dem Auflagebereich (3) aufgelegten Körperteils eingerichtet ist, optional ferner umfassend eine der Erfassungseinrichtung (12 - 15) zuordenbare oder zugeordnete, insbesondere in den Auflagebereich (3) und/oder den Auflagekörper (2) integrierte, Übertragungseinrichtung (16), welche zur Übertragung einer eine vermittels der Erfassungseinrichtung (23) erfasste chemische und/oder physikalische Größe beschreibenden Erfassungsinformation an wenigstens einen Kommunikationspartner (29) eingerichtet ist; und/oder
eine, insbesondere in den Auflagebereich (3) und/oder den Auflagekörper (2) integrierte, Erfassungseinrichtung (12), welche zur Erfassung eines auf den Auflagebereich (3) aufgelegten Körperteils eingerichtet ist; und/oder.
eine, insbesondere in den Auflagebereich (3) und/oder den Auflagekörper (2) integrierte, Erfassungseinrichtung (14), welche zur Erfassung wenigstens einer physiologischen Größe, insbesondere des Blutdrucks, der Sauerstoffsättigung oder des Pulses, eines ein Körperteil auf den Auflagebereich (3) auflegenden Nutzers eingerichtet ist; und/oder eine, insbesondere in den Auflagebereich (3) und/oder den Auflagekörper (2) integrierte, Erfassungseinrichtung (15), welche zur Erfassung einer Bewegung des Auflagekörpers (2) in wenigstens einem Bewegungsfreiheitsgrad, insbesondere relativ zu einem Untergrund, und/oder zur Erfassung einer Bewegung eines auf dem Auflagebereich (3) aufgelegten Körperteils in wenigstens einem Bewegungsfreiheitsgrad, insbesondere relativ zu dem Auflagekörper (2), eingerichtet ist.

9. Auflageeinrichtung nach einem der vorhergehenden Ansprüche, **gekennzeichnet durch** eine, insbesondere in den Auflagebereich (3) und/oder den Auflagekörper (2) integrierte, Datenspeichereinrichtung (17), welche zur Speicherung wenigstens einer vermittels der Erfassungseinrichtung (12 - 15) erfasster Erfassungsinformation eingerichtet ist.

10. Auflageeinrichtung nach einem der vorhergehenden Ansprüche, **gekennzeichnet durch** eine, insbesondere in den Auflagebereich (3) und/oder den Auflagekörper (2) integrierte, Signalausgabeeinrichtung (24), welche, insbesondere auf Grundlage wenigstens einer seitens der oder einer Erfassungseinrichtung (7, 9, 10, 11) erzeugten Erfassungsinformation, zur Erzeugung wenigstens eines, insbesondere akustischen und/oder optischen und/oder haptischen, Ausgabesignals eingerichtet ist.

11. Auflageeinrichtung nach einem der vorhergehenden Ansprüche, **gekennzeichnet durch** eine, insbesondere in den Auflagebereich (3) und/oder den Auflagekörper (2) integrierte, Temperiereinrichtung (16) zur zumindest abschnittsweisen, gegebenenfalls vollständigen, Temperierung des Auflagebereichs (3) und/oder des Auflagekörpers (2) und/oder eines auf der Auflageeinrichtung (1) aufzulegenden oder aufgelegten Körperteils; und/oder eine, insbesondere in den Auflagekörper (2) und/oder in den Auflagebereich (3) integrierte, Belüftungseinrichtung (22) zur zumindest abschnittsweisen, gegebenenfalls vollständigen, Belüftung des Auflagebereichs (3) und/oder des Auflagekörpers (2) und/oder eines auf der Auflageeinrichtung (1) aufzulegenden oder aufgelegten Körperteils.

12. Auflageeinrichtung nach einem der vorhergehenden Ansprüche, **gekennzeichnet durch** eine Fixiereinrichtung zur zeitweisen Fixierung eines auf den Auflagebereich (3) aufgelegten Körperteils, insbesondere in einer bestimmungsgemäßen Auflageposition und/oder Auflageausrichtung des Körperteils auf dem Auflagebereich (3).

13. Auflageeinrichtung nach einem der vorhergehenden Ansprüche, **gekennzeichnet durch** eine Befestigungseinrichtung (23) zur zeitweisen Befestigung der Auflageeinrichtung (1) an oder auf einem Drittgegenstand, insbesondere an oder auf einem an einem Körper eines Nutzers der Auflageeinrichtung (1) zu tragenden Drittgegenstands.

14. Auflageeinrichtung nach einem der vorhergehenden Ansprüche, **dadurch gekennzeichnet, dass** der Auflagekörper (2) wenigstens einen Lagerungsabschnitt (25) zur Lagerung des Auflagekörpers (2) auf einem Untergrund aufweist, wobei der Lagerungsabschnitt (25) wenigstens ein, insbesondere nicht plan auf einem planen Untergrund aufliegendes, Lagerungsabschnittelement (26) aufweist, welches eine in wenigstens einem Bewegungsfreiheitsgrad bewegbare, insbesondere schwenkbewegbare, Lagerung des auf dem Untergrund gelagerten Auflagekörpers (2) relativ zu dem Untergrund ermöglicht.

15. Verfahren zur Herstellung einer Aufnahmeeinrichtung (1) zur zumindest abschnittsweisen, gegebenenfalls vollständigen, Auflage wenigstens eines Körperteils eines Nutzers, umfassend die Schritte:
- Bereitstellen einer die Abmessungen eines auf einer herzustellenden Auflageeinrichtung (1) aufzulegenden Körperteils zumindest abschnittsweise, gegebenenfalls vollständig, beschreibenden Körperteilabmessungsinformation;
- Bereitstellen mehrerer Auflageeinrichtungsabmessungsinformationen, wobei jede Auflageeinrichtungsabmessungsinformation wenigstens eine wenigstens einer Abmessung eines Körperteils zugeordnete vordefinierte Abmessung einer auf Grundlage der jeweiligen Auflageeinrichtungsabmessungsinformation herzustellenden oder herstellbaren Auflageeinrichtung (1) beschreibt;
- Auswählen einer Auflageeinrichtungsabmessungsinformation auf Grundlage einer bereitgestellten Körperteilabmessungsinformation;
- Herstellen einer Auflageeinrichtung (1) auf Grundlage der ausgewählten Auflageeinrichtungsabmessungsinformation; wobei
optional das Auswählen durch Abgleich oder Vergleich der bereitgestellten Körperteilabmessungsinformation mit jeweiligen jeweiligen Auflageeinrichtungsinformationen zugeordneten Körperteilabmessungsinformationen ausgewählt wird, wobei eine Auflageeinrichtungsinformation ausgewählt wird, deren zugeordnete Körperteilabmessungsinformation der bereitgestellten Körperteilabmessungsinformation gleicht oder, insbesondere im Rahmen eines vordefinierbaren oder vordefinierten Toleranzbereichs, ähnelt.
